(19) **Europäisches Patentamt European Patent Office Office européen des brevets**

(11) **EP 4 380 426 B1**

(12) **EUROPEAN PATENT SPECIFICATION**

(45) Date of publication and mention
of the grant of the patent:
**18.03.2026   Bulletin 2026/12**

(21) Application number: **22737931.0**

(22) Date of filing: **22.06.2022**

(51) International Patent Classification (IPC):
***A61B 3/16*** *(2006.01)*

(52) Cooperative Patent Classification (CPC):
**A61B 3/16**

(86) International application number:
**PCT/FI2022/050451**

(87) International publication number:
**WO 2023/012402 (09.02.2023 Gazette 2023/06)**

(54) **METHOD AND APPARATUS FOR DETERMINING PHYSIOLOGICAL PARAMETER**

VERFAHREN UND VORRICHTUNG ZUR BESTIMMUNG PHYSIOLOGISCHER PARAMETER

MÉTHODE ET APPAREIL DE DÉTERMINATION D'UN PARAMÈTRE DE COMMUNICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   **05.08.2021   FI 20215838**

(43) Date of publication of application:
**12.06.2024   Bulletin 2024/24**

(73) Proprietor: **Icare Finland Oy**
**01510 Vantaa (FI)**

(72) Inventors:
• **EHNHOLM, Gösta**
**00120 Helsinki (FI)**
• **RAUDASOJA, Matti**
**01510 Vantaa (FI)**
• **HONKANEN, Viktor**
**01510 Vantaa (FI)**
• **KANGAS, Lauri**
**01510 Vantaa (FI)**
• **HERRANEN, Teemu**
**01510 Vantaa (FI)**
• **SALKOLA, Mika**
**01510 Vantaa (FI)**

(74) Representative: **Moosedog Oy**
**Kurjenmäenkatu 10 B 49**
**20700 Turku (FI)**

(56) References cited:
**US-A1- 2008 103 381     US-A1- 2019 380 577**

## Description

TECHNICAL FIELD

The present disclosure relates generally to ophthalmic treatment device; and more specifically, to methods and systems for determining physiological parameter.

BACKGROUND

Aqueous humor is a transparent water-like fluid produced in the eye to provide protection to the lens and maintains fluid pressure in the eye. The fluid pressure in the eye is known as intra-ocular pressure. Furthermore, the intra-ocular pressure is a physiological parameter that determines the strength of the eye, and find signs of optic nerve damage that might affect vision. Typically, the normal pressure in the eyes changes during the day and differ from person to person. However, in case the intra-ocular pressure is consistently too high or too low, there may be problems with the vision.

Traditionally, a tonometry test measures the intra-ocular pressure. Herein, the tonometry test uses a tonometer to determine the firmness of the eye. Conventionally, Goldman Applanation Tonometer (GAT) uses numbing drops to anesthetize the eyes, wherein a small amount of non-toxic dye is placed in the eye. Subsequently, a small probe gently touches the surface of the eye and the intra-ocular pressure is determined. Herein, the intra-ocular pressure is measured based on the force required to gently flatten a fixed area of the surface of the eye. However, the thickness of the eye, elasticity of the eye, and the amount of non-toxic dye used is a source of error and affects the accuracy of measurements obtained. Alternatively, Perkins Applanation Tonometer (PAT) is also used to measure the intra-ocular pressure. Herein, the PAT is a hand-held tonometer which is ergonomic and easy to use. However, the PAT requires a high level of skill to operate, decrease in stability as the PAT is a hand-held tonometer, and the need for topical application of fluorescein and anaesthetic which may lead to scarring of the surface of the eye. Furthermore, Tonopen is a form of rebound tonometry to measure the intra-ocular pressure. However, the Tonopen requires numbing drops to anaesthetize the eyes. The Tonopen further comprises a probe that bounces off of the surface of the eye in order to measure the intra-ocular pressure of the eye. Herein, the probe used is conical in structure. Furthermore, the conical structure may accidentally lead to a small scarring on the surface of the eye.

Therefore, in light of the foregoing discussion, there exists a need to overcome the aforementioned drawbacks associated with conventional methods related to measurement of physiological parameters.

US 2008/103381 A1 discloses:

- a probe formed of a non-magnetic front part and a magnetic or a magnetizable rear shaft part,
- a front coil which is used for moving the probe in one direction, wherein a voltage fed to the front coil induces a pushing force in the probe, causing it to move towards the eye,
- two coils of the solenoid type around the probe, one of the coils being used for moving the probe in one direction while the other coil being used to measure the movement of the probe,
- a microprocessor used to control the switches feeding the voltage to the coils
- the rear coil being configured to detect the speed of the probe continuously and to measure the movement of the probe.

US 2019/380577 A1 discloses:

- a disposable probe with an elongated shaft, at least a portion of which is made of a magnetic material, and a rounded tip,
- a conductive drive coil in which the probe is received,
- a controller configured to momentarily energize the drive coil to propel the probe forward toward the eye by electromagnetic force,
- a conductive measurement coil through which the probe moves,
- a controller configured to measure a current induced in the measurement coil by the moving probe and provide a measurement signal representing velocity of the probe as a function of time.

SUMMARY

The present disclosure seeks to provide an apparatus for determining physiological parameter. The present disclosure also seeks to provide a method for determining a physiological parameter. An aim of the present disclosure is to provide a solution that overcomes at least partially the problems encountered in prior art.

In one aspect, the present disclosure provides an apparatus for determining physiological parameter, the apparatus comprising:

- an elongated magnetic probe, having a first end, a second end opposite to the first end and a middle section between the first end and the second end;
- a driver coil arranged partially to surround the elongated magnetic probe;
- a measurement coil comprising at least a first section and a second section, and the measurement coil arranged partially to surround the elongated magnetic probe and
- a controller configured to

  - selectively energize the driver coil to create a magnetic force to initiate movement of the elongated magnetic probe to a direction of the first end, wherein the elongated magnetic probe is directed to hit a surface of the eye and bounce back thereof;
  - measure first induced voltage values over the first section and common induced voltage values over at least one of: the first section and the second section, or over the second section, as a function of time during a time of the movement of the elongated magnetic probe;
  - determine locator values as a function of time by dividing the first induced voltage values with the common induced voltage values;
  - map the locator values as a function of time from the time domain to a spatial domain and
  - calculate from the spatial domain locator values, a first velocity profile of the elongated magnetic probe, and use the calculated first velocity profile of the elongated magnetic probe to determine the physiological parameter.

In another aspect, the present disclosure provides a method for determining a physiological parameter, the method using a controller to carry out the method steps comprising:

- energizing a driver coil to move elongated magnetic probe to a direction of a first end of the elongated magnetic probe, wherein the elongated magnetic probe is directed to hit a surface of the eye and bounce back thereof;

- measuring as a function of time, over a first section of a measurement coil, an induced first voltage;

- measuring as a function of time, over at least one of: a first section and a second section or over the second section of the measurement coil, an induced common voltage;

- determining a first locator value as a function of time by dividing the measured induced voltage values with measured induced common voltage values;

- mapping the first locator values from time domain to a spatial domain;

- calculating from the spatial domain first locator values a first velocity profile of the elongated magnetic probe and

- using the first velocity profile to determine the physiological parameter.

Embodiments of the present disclosure substantially eliminate or at least partially address the aforementioned problems in the prior art, and enable to accurately determine the position of the elongated magnetic probe by calculating the induced voltages

Additional aspects, advantages, features and objects of the present disclosure would be made apparent from the drawings and the detailed description of the illustrative embodiments construed in conjunction with the appended claims that follow.

It will be appreciated that features of the present disclosure are susceptible to being combined in various combinations without departing from the scope of the present disclosure as defined by the appended claims.

BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the present disclosure is not limited to specific methods and instrumentalities disclosed herein. Moreover, those skilled in the art will understand that the drawings are not to scale. Wherever possible, like elements have been indicated by identical numbers.

Embodiments of the present disclosure will now be described, by way of example only, with reference to the following diagrams wherein:

FIG. 1                   is a block diagram of a system for an apparatus for determining physiological parameter, in accordance with an implementation of the present disclosure;

FIG. 2                   is a graph to depict a force on elongated magnetic probe induced per ampere of current in signal or drive coil versus position in millimeters,

FIG. 3                   is a graph to represent the common induced voltage of the measurement coil,

FIG. 4                   is a illustration of measurement of three locator signals using four differential amplifiers to measure the voltages across each of the signal coil segments,

FIG. 5                   is a graph to determine the position of the elongated magnetic probe, in accordance with an implementation of the present disclosure;

FIG. 6                   is an assembly of the measurement coil, in accordance with an implementation of the present disclosure;

FIG. 7                   is a schematic illustration view of a system for a robotic system to determine the speed and position of the elongated magnetic probe, in accordance with an implementation of the present disclosure;

FIG. 8                   is an electric circuit to add the induced voltages, in accordance with an implementation of the present disclosure;

FIG. 9                   is induced signal voltages in the measurement coil, in accordance with an implementation of the present disclosure,

FIG. 10                  is a block diagram for a system for basic implementation of the apparatus, in accordance with an implementation of the present disclosure;

FIGs. 11A and 11B        are graphs to realize an optimal connection for the first amplifier and the second amplifier, in accordance with an implementation of the present disclosure;

FIGs. 12A and 12B        are graphs to illustrate signal voltages and common induced voltage calues, in accordance with an implementation of the present disclosure;

FIGs. 13A to 13D         collectively are graphs to measure the locator values by the addition of plurality of signal amplifiers, in accordance with an implementation of the present disclosure;

FIGs. 14A and 14B        collectively illustrate a flowchart depicting steps of a method for determining a physiological parameter, in accordance with an embodiment of the present disclosure and

FIG 15                   is an illustration of determining velocity profile.

In the accompanying drawings, an underlined number is employed to represent an item over which the underlined number is positioned or an item to which the underlined number is adjacent. A non-underlined number relates to an item identified by a line linking the non-underlined number to the item. When a number is non-underlined and accompanied by an associated arrow, the non-underlined number is used to identify a general item at which the arrow is pointing.

DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practising the present disclosure are also possible.

In one aspect, an embodiment of the present disclosure provides a An apparatus for determining physiological parameter, the apparatus comprising;

- an elongated magnetic probe, having a first end, a second end opposite to the first end and a middle section between the first end and the second end;
- a driver coil arranged partially to surround the elongated magnetic probe;
- a measurement coil comprising at least a first section and a second section, and the measurement coil arranged partially to surround the elongated magnetic probe and
- a controller configured to

  - selectively energize the driver coil to create a magnetic force to initiate movement of the elongated magnetic probe to a direction of the first end, wherein the elongated magnetic probe is directed to hit a surface of the eye and bounce back thereof;
  - measure first induced voltage values over the first section and common induced voltage values over at least one of: the first section and the second section, or over the second section, as a function of time during a time of the movement of the elongated magnetic probe;

- determine locator values as a function of time by dividing the first induced voltage values with the common induced voltage values;
- map the locator values as a function of time from the time domain to a spatial domain and
- calculate from the spatial domain locator values, a first velocity profile of the elongated magnetic probe, and use the calculated first velocity profile of the elongated magnetic probe to determine the physiological parameter.

Preferably the measurement of the voltage values from the first section is done simultaneously during measurement of the common induced voltage. Common induced voltage can be measured over a first section and a second section (i.e. voltage of a first section added to voltage of the second section) or as voltage over the second section. The second section can overlap the first section. In deed one can use several sections of measurement coil and combine their signal voltages in different ways before calculating locator signals that are independent of probe velocity. In an optional embodiment the measurement of first section is done first and the on the other measurement cycle the measurement of the common induced voltage is done. A common start of relative time base (t0) is defined as start of the measurement for both separately.

In another aspect, an embodiment of the present disclosure provides a method for determining a physiological parameter, the method using a controller to carry out the method steps comprising

- energizing a driver coil to move elongated magnetic probe to a direction of a first end of the elongated magnetic probe, wherein the elongated magnetic probe is directed to hit a surface of the eye and bounce back thereof;

- measuring as a function of time, over a first section of a measurement coil, an induced first voltage;

- measuring as a function of time, over at least one of: a first section and a second section or over the second section of the measurement coil, an induced common voltage;

- determining a first locator value as a function of time by dividing the measured induced first voltage values with respective measured induced common voltage values;

- mapping the first locator values from time domain to a spatial domain;

- calculating from the spatial domain first locator values a first velocity profile of the elongated magnetic probe and

- using the first velocity profile to determine the physiological parameter.

Moreover, velocity and magnetization of the elongated magnetic probe is determined, thereby making the measurements more accurate.

The present disclosure provides an apparatus for determining physiological parameter. Herein the apparatus refers to an instrument that is used to measure a physiological parameter of the eye such as, intra-ocular pressure of the eye, touch sensitivity and the like. In an embodiment, the apparatus is a tonometer. Herein, the tonometer is used for measuring intra-ocular pressure from an ophthalmic measurement. Furthermore, the intra-ocular pressure is calculated from a voltage signal representing velocity as a function of time of an elongated magnetic probe rebounded from the surface of an eye. The magnitude of the signal depends on the magnetic strength and speed of the elongated magnetic probe.

The apparatus comprises an elongated magnetic probe, having a first end, a second end opposite to the first end and a middle section between the first end and the second end. Herein, the first end of the elongated magnetic probe is made from bio-compatible material and will collide with surface of the eye when in use. Beneficially, the first part being made of bio-compatible material enables the probe to function in intimate contact with living tissues of the eye causing minimal discomfort or pain. Notably, the bio-compatible material is free from carcinogenicity, toxicity, and is resistive to corrosion. Furthermore, the elongated magnetic probe may be made of thin wire of magnetic material. Herein, the elongated magnetic probe may be for instance 20 millimeters in length and 0.5 millimeter in width. Additionally, the magnetic material in the elongated magnetic probe may be ferromagnetic. Moreover, the movement of the elongated magnetic probe inside a measurement coil produces an induced voltage when it moves. However, the magnetic forces of the elongated magnetic probe are insignificantly small, wherein the elongated magnetic probe is pushed back only by the surface of the eye, with no other force being present.

The apparatus comprises a driver coil arranged partially to surround the elongated magnetic probe. Herein, the driver coil is arranged as a loop through which the elongated magnetic probe can move. Furthermore, the driver coil has finite number of loops. Additionally, the driver coil may be arranged along any point between the first end and the second end. For instance, the driver coil may be arranged closer to the first end. Subsequently, the driver coil moves the elongated magnetic probe when electric current is fed through the driver coil. Furthermore, the driver coil pulls the elongated magnetic probe and projected towards the surface of the eye with a velocity which is equal to product of the electric current fed through the driver coil times the elongated magnetic probe magnetization.

The apparatus comprises a measurement coil comprising at least a first section and a second section, and the measurement coil arranged partially to surround the magnetic probe. Herein, the measurement coil has a finite number of loops. Furthermore, the total number of loops of the measurement coil are divided between the first section and the second section. Herein, each of the first section and the second section may have at least one loop through which the elongated magnetic probe is able to move. Furthermore, the elongated magnetic probe may move along the middle section, wherein the middle section is between the first section and the second section. Since the measurement coil comprises two or more sections it is possible to measure at the same time different voltage profiles induced by moving elongated magnetic probe. Technical effect of this is to eliminate uncertainty of magnetization constant of the probe. Indeed, as will be discussed later, making the measurements with at least two different measurement coils allows determination of the velocity of the probe even if the magnetization value is not known at all.

Optionally, the measurement coil is used during first period of time as the driver coil and during a second period of time, which the second period of time is after the first period of time, as the second section of the measurement coil. Herein, the measurement coil is used to drive the electric current, thereby inducing a motion to the elongated magnetic probe. Subsequently, immediately after the elongated magnetic probe is accelerated to its operational velocity, the measurement coil is used to measure induced voltage to the measurement coil. Beneficially, the number of coils in the apparatus is reduced. Furthermore, the measurement coil may have multiple functions depending on the manner a controller controls the measurement coil, such as for example whether electric current is driven or induced voltage is measured.

Optionally, the first section and the second section of the measurement coil are connected in series. Herein, series connection refers to an electrical coupling of the last loop of the first section to the first loop of the second section. Typically, measuring of voltages induced to the measurement coils in a series connection can be realized by connecting a voltmeter to the first section of the measurement coil and the second section of the measurement coil. Alternatively, the induced voltage over the first section is measured. Subsequently, the induced voltage over both the first section and the second section may be measured together to derive total induced voltage value. The voltage value over the second section can be calculated by subtracting from the total induced voltage value of the induced voltage to the first section.

Optionally, the measurement coil comprises a third section, the third section connected in series with the first and the second section of the measurement coil and the third section is arranged to surround a third section of the elongated magnetic probe. Herein, the third section may have at least one loop through which the elongated magnetic probe is able to move. Furthermore, multiple sections may be added in the measurement coil, thereby generating more data points.

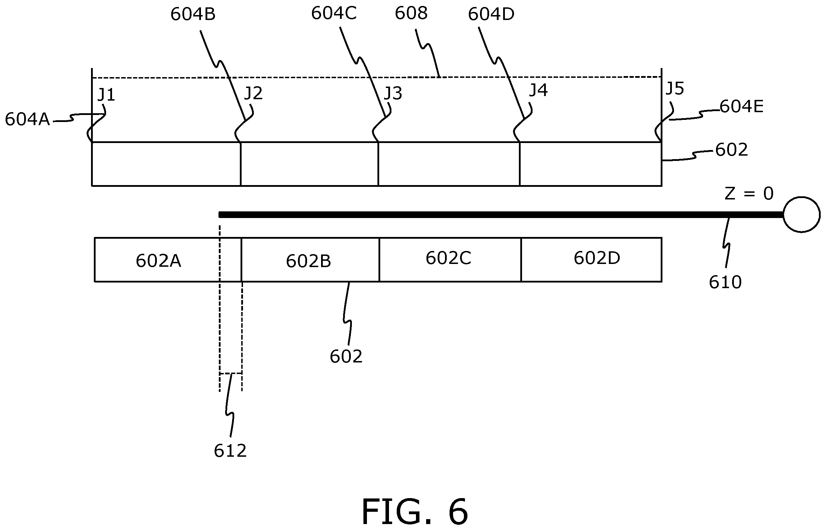

In an example, the measurement coil is divided into the first section, the second section, the third section and the fourth section, and the driver coil comprises one section. Herein, a plurality of coupling leads are wired to the points connecting the first section, the second section, the third section and the fourth section of the measurement coil to each other. Furthermore, a first coupling lead is connected to the leftmost end of the first section, a second coupling lead is connected to the junction of the first section and the second section, a third coupling lead is connected to the junction of the second section and the third section, a fourth coupling lead is connected to the junction of the third section and the fourth section, and a fifth coupling lead is connected to the rightmost end of the fourth section. Such exemplary implementation of the measurement coil is further explained in detail in FIG. 6.

The apparatus comprises a controller. Herein, the controller is a computational device that is operable to to respond to and process information. In an example, the controller may be an embedded microcontroller, a microprocessor, computer or a portable computing device. Furthermore, the controller is communicably coupled with the measurement coil and the driver coil. Additionally, the controller energizes the driver coil to move the elongated magnetic probe towards the surface of the eye.

The controller is configured to selectively energize the driver coil to create a magnetic force to initiate movement of the elongated magnetic probe to a direction of the first end. Herein, selectively energizing refers to switching the supply voltage of the driver coil ON or OFF. Notably, an electric field is created when the supply voltage of the driver coil is switched ON. Typically, higher supply voltage of the driver coil will result in greater magnetic force. Subsequently, the acceleration of the elongated magnetic probe will increase. Moreover, the magnetic force is a function of magnetization of the elongated magnetic probe. Herein, the magnetization refers to a strength of magnetisation of the elongated magnetic probe. In particular, higher magnetization will result in greater magnetic force. Additionally, selectively energizing the driver coil will move the elongated magnetic probe towards the surface of the eye. Moreover, the elongated magnetic probe will move in an alternate direction in case polarity of the driver coil by selectively energizing is reversed. Herein, the elongated magnetic probe moves in a direction which may be controlled by the controller whenever required, wherein the voltage is a function of speed of the elongated magnetic probe and magnetization of the elongated magnetic probe. Additionally, the speed of the elongated magnetic probe is monitored continuously by the measurement coil as a function of time. Subsequently, such information relating to the speed of the elongated magnetic probe may be used for determining pressure of the eye which may be used for diagnostic purposes. Typically, a magnetic field is induced in the driver coil when the electric current is provided to the driver coil. Furthermore, the magnetic field is proportional to the electric current and the finite number of loops of the driver coil. Typically, the magnetic field can be controlled by controlling the electric current provided to the driver coil. Subsequently, the magnetic field causes the magnetic force on the elongated magnetic probe, wherein the magnetic force is a function of magnetization of the elongated magnetic probe. Furthermore, acceleration of the elongated magnetic probe can be calculated by dividing mass of the elongated magnetic probe by the force on the elongated magnetic probe. Henceforth, the elongated magnetic probe is accelerated by current pulse in the driver coil, thereby generating a magnetic field acting on the elongated magnetic probe. Furthermore, the elongated magnetic probe is sent through the measurement coil towards the surface of the eye, from which the elongated magnetic probe bounces back. One technical problem of performing above measurements is uncertainty on value of magnetization. Indeed if we compare two probes from different manufacturing patches there can be significant difference between magnetization values or capability to be magnetization of the probes. This would lead to uncertainty when measuring the induced voltages. As an example same induced driver voltage (current) will result to faster movement of the probe as function of magnetization value of the probe.

Optionally, a magnetization cycle can be integrated in the present disclosure for magnetization of the elongated magnetic probe. Herein, the magnetization cycle is achieved by current pulse in the driver coil and the measurement coil respectively, thereby pulling the elongated magnetic probe back and forth in the apparatus. Furthermore, the magnetization cycle may end with a calibration cycle. Herein, the calibration cycle collects the information required to set the acceleration in the driver coil to a value for obtaining an optimal speed for the elongated magnetic probe. Furthermore, the calibration cycle checks both the apparatus and the elongated magnetic probe, before determining the physiological parameter. In one embodiment the calibration cycle can be used to collect lookup table values for mapping collator values as a function of time from the time domain to a spatial domain.

In an embodiment, when in use, the first section of the measurement coil surrounds a first section of the elongated magnetic probe and the second section of the measurement coil surrounds a second section of the elongated magnetic probe, wherein the first section of the elongated magnetic probe is different from the second section of the elongated magnetic probe, when the elongated magnetic probe is in its first spatial position. Herein, the geometrical setup of the apparatus wherein first spatial position is initial position before the elongated magnetic probe is selectively energized. Furthermore, in the first position the elongated magnetic probe is retracted fully inside the apparatus.

Optionally, when in use, the first section of the measurement coil does not surround the first section of the elongated magnetic probe and the second section of the measurement coil surrounds the second section of the elongated magnetic probe, when the elongated magnetic probe is in its second spatial position, which the second spatial position is different from the first spatial position. Herein, the second spatial position may be for example when the elongated magnetic probe is moved to an extreme position, for instance, almost out of the measurement coil. Furthermore, as the first section of the measurement coil does not surround the elongated magnetic probe at all, the change in magnetic flux on the measurement coil is greater as compared to the measurement coil surrounding the elongated magnetic probe partially. Moreover, the magnetic flux is constant for an evenly magnetized elongated magnetic probe throughout. Herein, magnetic flux lines leave from one end, and re-enter from the opposite end. Furthermore, some of the magnetic flux lines pass through the wall of the measurement coil while re-entering from the opposite end. Moreover, the elongated magnetic probe is long and fits closely to the measurement coil so that the magnetic flux in each turn of the measurement coil changes with time.

The electric current in the measurement coil acts on the elongated magnetic probe with a controlled magnetic field pulse, thereby sending the elongated magnetic probe with the desired speed to the surface of the eye. Herein, the speed of the elongated magnetic probe is determined by the amplitude of the current pulse, length and the magnetization of the elongated magnetic probe, and friction between the elongated magnetic probe with the measurement coil and the driver coil respectively. Furthermore, the elongated magnetic probe bounces back when it hits the surface of the eye. Moreover, the intra-ocular pressure can be determined by measuring the speed profile of the elongated magnetic probe, while the elongated magnetic probe is approaching, rebounding, and or returning.

The controller is configured to measure first induced voltage values from the first section and common induced voltage values, as a function of time during a time of the movement of the elongated magnetic probe. Herein, when the elongated magnetic probe moves inside the loops of the first section and the second section, voltage is induced as per Faraday's law of induction. Furthermore, the controller is configured to measure the first induced voltage value as function of time during the movement of the elongated magnetic probe over the first section. Additionally, the common induced voltage of the measurement coil as a function of time is measured. The common induced voltage in case of having two sections (the first section and the second section) is induced voltage over both sections. If there are for example three or four sections the common induced voltage is induced voltage over first, second, third and four sections.

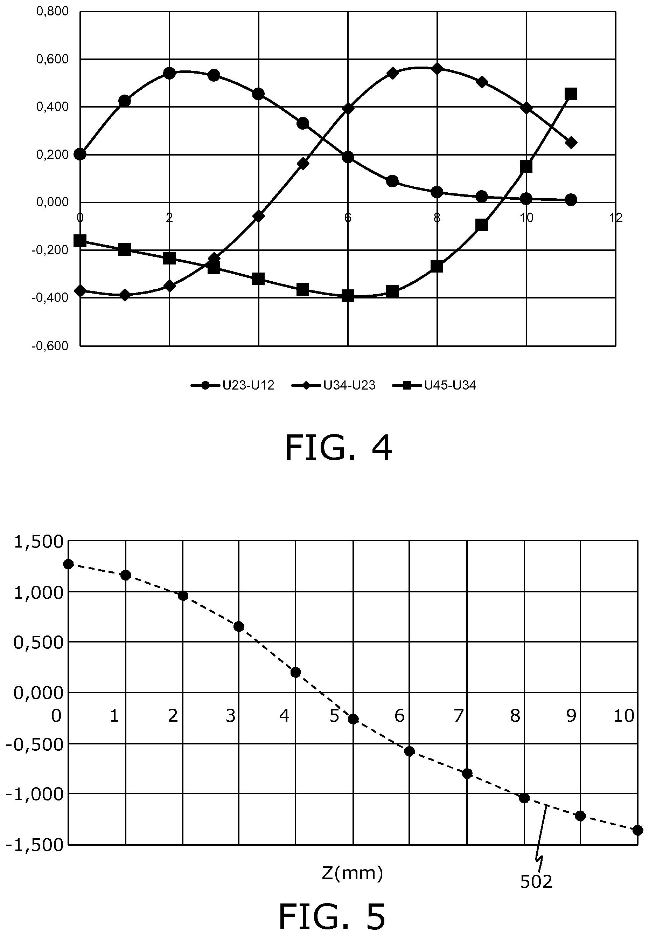

Additionally, optionally, the sampling interval of the induced voltage and the geometry of the measurement coil are controlled by controlling the position of the elongated magnetic probe precisely using the robotic system. Moreover, as the elongated magnetic probe passes through the first section, the second section, the third section and the fourth section towards the surface of the eye, the induced voltage changes as the function of time. Furthermore, the amplitudes of the induced voltage are proportional to the position and velocity of the elongated magnetic probe. Additionally, the common induced voltage is suited for measuring the speed, when the magnetization of the elongated magnetic probe is known. This has been explained further in detail in conjunction with FIG. 4. As discussed one technical problem is that the magnetization of the elongated magnetic problem can have a range of values thus making straightforward calculation of the speed difficult.

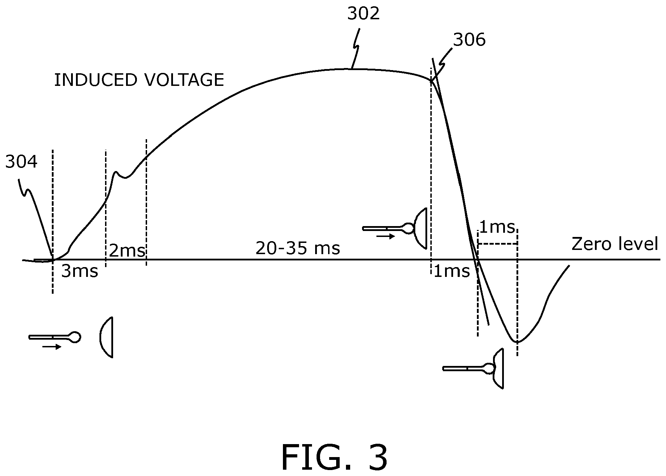

Initially, the common induced voltage (and the first induced voltage) increases during the acceleration provided by the driver coil to the elongated magnetic probe. Subsequently, the elongated magnetic probe hits the surface of the eye and decelerates for about 1 millisecond (ms) to zero after from 20 milliseconds up to 35 millisecond and then bounces back, depending on the exact velocity and distance from the surface of the eye. The deceleration of the elongated magnetic probe may for example be carried out at 20, 24, 28 or 32 milliseconds up to 21, 25, 30 or 35 milliseconds. Herein, the trajectory of the speed of the elongated magnetic probe during contact with the surface of the eye is mainly dependent on the speed of the elongated magnetic probe and, via a pushback force, or intra-ocular pressure. Furthermore, the main parameter for dependence on the intra-ocular pressure is given by the slope of deceleration of the speed of the elongated magnetic probe, wherein deceleration of the speed of the elongated magnetic probe is equal to the magnetic force between the elongated magnetic probe and the surface of the eye. Additionally, the apparatus of the present disclosure provides with some corrections, an output signal from the elongated magnetic probe from which the intra-ocular pressure is derived. Notably, the output signal is verified with patient data, using other types of measurements, to generate a curve to translate to reveal the true intra-ocular pressure. Consequently, with the exception at low intra-ocular pressures, the intra-ocular pressure is equal to approximately a factor of 1.5 times the input signal minus a constant. Moreover, the factor becomes larger and the constant becomes smaller at low intra-ocular pressures. This has been explained further in detail in conjunction with FIG. 3. Furthermore, the first signal amplifier and the second signal amplifier used are operational amplifiers. Herein, the operational amplifiers add voltages at the input of amplifying stage via the resistors to the negative and positive terminals of the first signal amplifier and the second signal amplifier. Furthermore, several induced voltage signals may be easily added or subtracted into a common output with weights set by the resistors. Specifically, a differential amplifier may be used for simple implementation of the present disclosure. Herein, the first input of the differential amplifier is connected to the negative terminal and the second input of the differential amplifier is connected to the positive terminal. Additionally, a basic implementation may be performed using a first amplifier and a second amplifier. Herein, the first amplifier has multiple inputs for addition of selected induced voltage from the first section or the second section or optionally, the third section or the fourth section. Furthermore, the second amplifier is a differential amplifier for calculating the common induced voltage. In an example, the first amplifier is electrically coupled with the second section and the third section, thereby determining the difference between the induced voltages of the second section and the third section. Furthermore, the second amplifier is electrically coupled with the first section and the fourth section, thereby generating the common induced voltage. In an example, the first induced voltage of the first section is referred to as 'V1', the second induced voltage of the second section is referred to as 'V2', and the third induced voltage of the third section is referred to as 'V3'. Furthermore, four resistors 'R1', 'R2', 'R3' and 'R4' are connected to the differential amplifier. In such example, the output voltage $V_{out}$ is calculated as,

$$V_{out} = \left( V1 \cdot \frac{R1}{R1 + R2} + V2 \cdot \frac{R1}{R1 + R2} \right) \left( 1 + \frac{R4}{R3} \right) - V3 \cdot \frac{R4}{R3}$$

It turns out that for detecting very small signals the method of using differerential amplifiers giving Vout proportional to V1-V3 is practical for removing interfering signals: Such amplifiers are commercially available for this purpose.

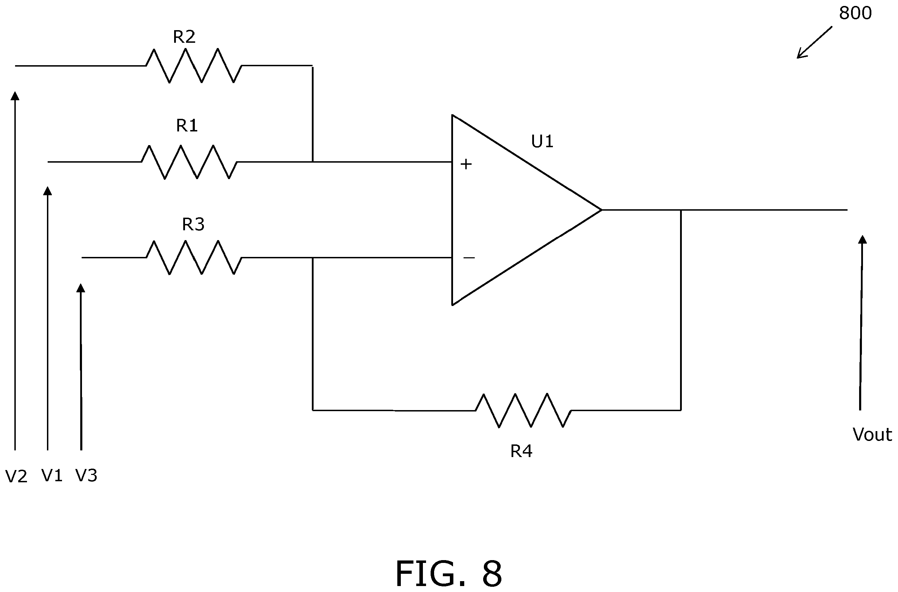

Such example is explained further in detail in conjunction with FIG. 8. Furthermore, any combination of induced voltages of the measurement coil can be obtained using an operational amplifier with four inputs or less, by adjusting the sign and amplification factors with the values of the summing resistors. Additionally, using a combination of the outputs of the first amplifier and the second amplifier, an optimized signal may be obtained. Herein, the optimized signal may be sensitised as per requirement, such as for example the position of the elongated magnetic probe, and unsensitized to another parameter, such as for example the degree of magnetization.

The controller is configured to determine locator values as a function of time by dividing the first induced voltage values with the common induced voltage values. Technical effect for dividing (pair wise) first voltage values with respective common induced voltage values is the eliminate unknown magnetization constant of the elongated magnetic probe. In deed introduced voltage is a function of change in magnetic flux $\Phi$ in a measurement coil. The magnetic flux is a function of magnetic field B i.e. magnetization of the elongated magnetic probe. Since B is constant it can be eliminated by dividing the first induced voltage with the common induced voltage values. The locator values refer to values which are correlated with the actual location of the elongated magnetic probe. Furthermore, the locator values are initially a function of time considering that the measurements are made as a function of time. Thereby, the locator values are in time domain.

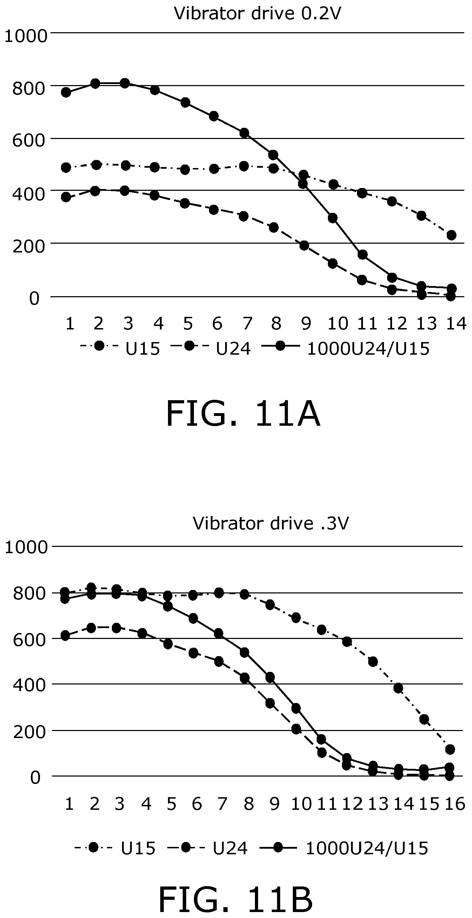

Furthermore, the first induced voltage value is divided with respective measurement of common induced voltage. Experimentally, division of the first induced voltage coil value with common induced voltage values provides similar formfactor. Consequently, the locator values are independent of the speed of the elongated magnetic probe. Beneficially, the speed of the elongated magnetic probe is the function of magnetization, thereby eliminating variations of electric current of the driver coil on probe magnetization. Typically, the first induced voltage values and the common induced voltage values are measured to solve uncertainty of the magnetization of the elongated magnetic probe. Furthermore, the first induced voltage values and the common induced voltage values are function of velocity and the magnetization of the elongated magnetic probe. In an example, the first signal amplifier measures the common induced voltage of the entire measurement coil for the 0.2 volts (V) driver coil and for a 0.3 V driver coil. Herein, the common induced voltage is referred to as '*U15*'. Furthermore, the second signal amplifier is electrically coupled with the second coupling lead of the second section and the fourth coupling lead of the junction of the third section and the fourth section, thereby referred to as '*U24*'. However, upon division of '*U24*' with '*U15*,* the result is exactly the same in the case of the 0.2V driver coil as well as the 0.3 V driver coil, by virtue of dependency on the amplitude of the driver coil being factored out. Particularly, the speed of the elongated magnetic probe directly affects the induced voltages. Herein, the second induced voltage values have increased by 1.6, and the locator values in the middle range are the same within the accuracy of the measurement. Furthermore, voltage signals below Z = 5 millimetres (mm) and above Z = 10 millimetres are not considered, as the induced voltage values have not changed rapidly as a function of Z and the locator values are constant. This has been explained further in detail in conjunction with FIG. 11.

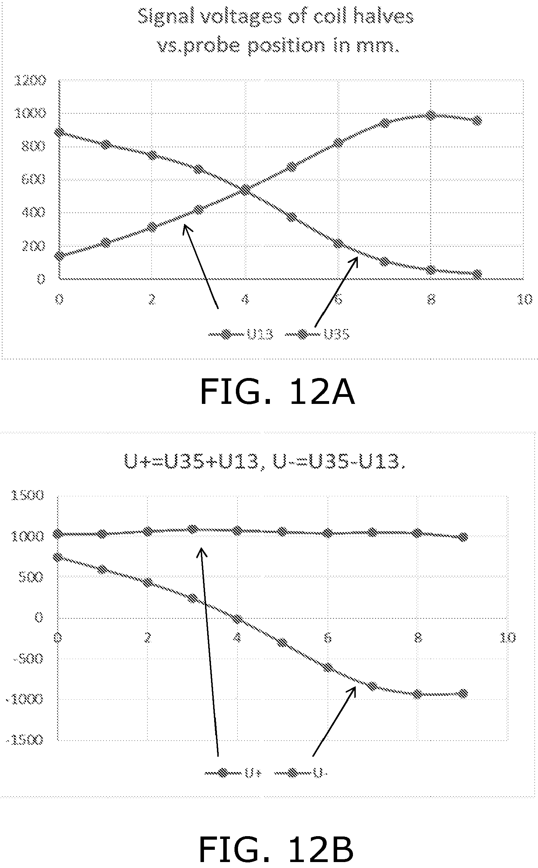

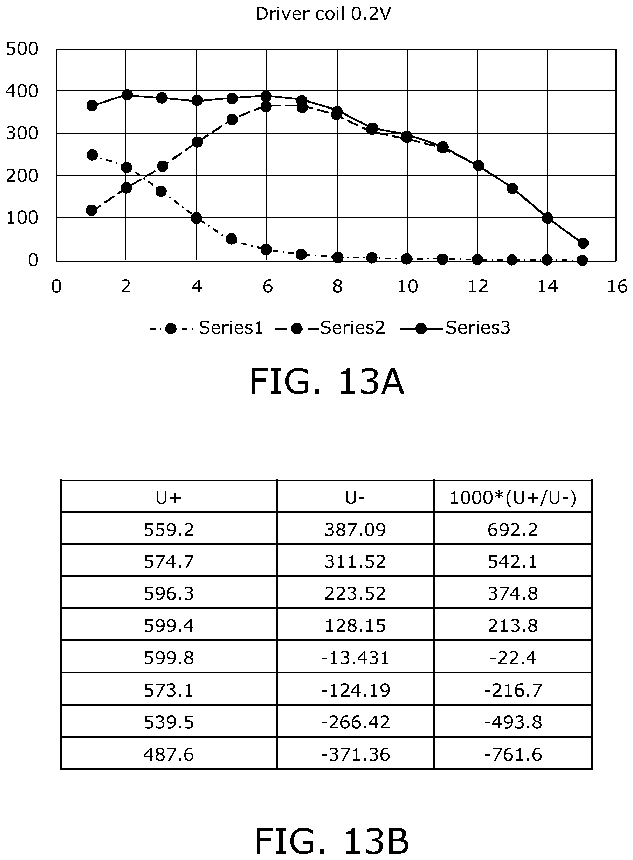

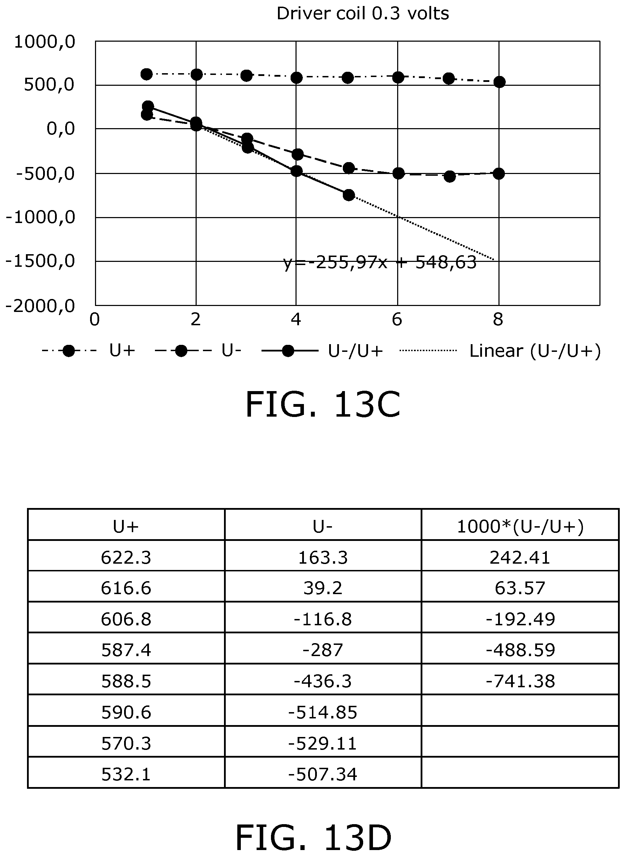

Continuing with the previous example, the useful range is mostly towards the left and becomes narrower. Thereby, the present disclosure may be implemented as per requirements. Herein, the useful range may be widened by using a fourth signal amplifier, a fifth signal amplifier and so forth. This has been explained further in detail in conjunction with FIG. 12. Fig. 13 shows the signals collected for the case of using two amplifiers in a way that covers a wide region centered to the region of interest. The U15 signal is measured by summing the output signals from the first signal amplifier and the second signal amplifier. Herein, the summation of the output signals from the first signal amplifier and the second signal amplifier is referred to as 'U+'. Furthermore, the difference between the output signal from the first signal amplifier and the second signal amplifier is referred to as 'U-'. Typically, the locator values are calculated by the ratio of the difference of the output signals from the first signal amplifier and the second signal amplifier by the sum between the output signals from the first signal amplifier and the second signal amplifier,

$$ L = \frac{U-}{U+} $$

The controller is configured to map the voltage U13 and U35 as a function of time. Herein, the time domain refers to the locator values as a function of sampling time. Furthermore, mapping may be executed by using, such as for example a lookup table, wherein the locator values are determined by experiments for each geometry of the measurement coil. Herein, this can be implemented, such as for example by moving the elongated magnetic probe back and forth with the actuator, and simultaneously measuring the induced voltage values. In view of the fact that the movement is done with the driver coil, relationship between the induced voltage values and spatial positions of the elongate magnetic probe can be determined.

Optionally, the time domain to spatial domain is mapped by at least one of: pre-determined transfer function or a look up table. The mapping of time domain signals is, for instance, done by placing the probe in exact locations and vibrating it with a very small displacement at a suitable frequency, of the order of 1 kilohertz. The time domain signal at this location is proportional to the induced voltage at 1 kHz. This is done in order to calibrate location curve. Mapping to the spatial domain from the time domain is needed step in order to determine velocity of the probe. Creation of the pre-determined transfer function is disclosed in example 1 below and example of how to determine a look up table is in example 2.

The controller is configured to calculate from the spatial domain locator values, a first velocity profile of the elongated magnetic probe, and use the calculated first velocity profile of the elongated magnetic probe to determine the physiological parameter. Herein, the first velocity profile is calculated from the locator values in the time domain against the locator values in the spatial domain. Moreover, depending on the target application, the velocity as the function of time is used to determine the physiological parameter, such as for example the intra-ocular pressure. In an example, the speed of the elongated magnetic probe and the response of the surface of the eye to stop the elongated magnetic probe once it is ejected towards the surface of the eye may be determined by calculating first derivate of the velocity. Furthermore, the speed of the elongated magnetic probe rebounding from the surface of the eye may be determined. Herein, the intra-ocular pressure is high in case the elongated magnetic probe rebounds rapidly, and the intra-ocular pressure is low in case the elongated magnetic probe rebounds slowly. Subsequently, the measurements regarding the time and velocity of the elongated magnetic probe may be collected with medical trials and function of speed of the elongated magnetic probe formfactors is compared with medical trial measurements as discussed in the present disclosure to determine physiological parameters. Furthermore, the speeds of the elongated magnetic probe may be determined by dividing the first induced voltage value to the second induced voltage value, thereby giving a relative value which is used to determine the speed of the elongated magnetic probe. Optionally, wherein the first velocity profile comprises at least one of: a velocity in the spatial domain, a velocity in the time domain. Optionally, the second velocity profile comprises at least one of: a velocity in the spatial domain, a velocity in the time domain.

Optionally the controller of the apparatus is further configured to

- measure a second induced voltage values from a section other than the first section, as a function of time during a time of the movement of the elongated magnetic probe;
- determine a second locator values as function of time by dividing the second induced voltage values with the common induced voltage values;
- map the second locator function from a time domain to a spatial domain;
- calculate from the spatial domain second locator values a second velocity profile of the elongated magnetic probe and
- use the calculated second velocity profile the elongated magnetic probe to update the physiological parameter. Updating can be done for example by calculating average between the first velocity profile and the second velocity profile and use the average as the velocity profile to determine physiological parameter.

The present disclosure also relates to the method as described above. Various embodiments and variants disclosed above apply *mutatis mutandis* to the method.

The elongated magnetic probe is directed to move towards a patient to hit a surface of the patient body and bounce back thereof. The surface refers to surface of eye of the patient. By directing the probe to surface and measuring velocity profile gives indication for example of eye pressure of the patient. If, for example, velocity profile indicates decreased acceleration when the probe hits the eye it is indication of low eye pressure. Higher acceleration (i.e. change of speed profile) can indicate higher eye pressure.

Optionally, the measurements are carried out during the movement of the elongated magnetic probe to obtain induced voltage values as function of time.

Optionally, the determination of the physiological parameter is carried out at least by one of: analysing acceleration of the elongated magnetic probe during its impact to the surface of the patient body, change of velocity before the impact and after the impact, amount of penetration of the elongated magnetic probe to the surface of the body. Acceleration can be determined from the velocity profile as the acceleration is derivate of the velocity in respect to time.

Optionally, the physiological parameter value is updated by

- measuring as a function of time, with a section of a measurement coil different from the first section, a second induced first voltage;
- determining a second locator values as function of time by dividing the second induced voltage values with the common induced voltage values;
- mapping the second locator function from a time domain to a spatial domain;
- calculating from the spatial domain second locator values, a second velocity profile of the elongated magnetic probe as function of location;
- using the calculated second velocity profile for updating the physiological parameter.

Optionally, the first velocity profile comprises at least one of: a velocity in the spatial domain, a velocity in the time domain.

Optionally, the second velocity profile comprises at least one of: the velocity in the spatial domain, the velocity in the time domain.

EXAMPLE 1: Creating of pre-determined transfer function by experiments:

Furthermore, mapping of locator values is executed to obtain the localization, that is the value of Z and velocity dZ/dt of the probe. Mathematically, the elongated magnetic probe is mapped into linear space with Z for the position, wherein to measure a first function L with respect to Z, a robotic system is used for setting the value of Z and for mapping. Herein, the first function L with respect to Z is referred to as *'L(Z)'*. Additionally, a second function L with respect to Z is determined such that the second function is dependent on position of the elongated magnetic probe, and is independent of the signal coil induced voltage signal, the degree of the elongated magnetic probe magnetization and the probe velocity. Moreover, time derivative of Z is used for calculating the velocity (v) of the elongated magnetic probe,

$$v=dZ/dt=(dL/dt)/dL/dZ)$$

For instance, for an elongated magnetic probe moving at fast speeds, the locator values are sampled with a short time interval, where in the short time interval is given by the formula $T_n$ - $T_n$ - 1. Herein, 'n' denotes a finite time interval. Additionally, the locator values will increase correspondingly, which is given by the formula, $L_n$ - $L_n$ - 1. Therefore, the velocity of the elongated magnetic probe for the $n^{th}$ sample is given by

$$v_n = \frac{K(L_n - L_n - 1)}{T_n - T_n - 1}$$

Furthermore, data points can be collected while making long sweeps of Z-values, starting at Z=0, and thereby making several steps each of 1 mm. Herein, the voltage signals are root mean square value of the measured voltage in millivolt (mVrms) at the output of the digital oscilloscope. Notably, the range of Z giving useful locator values is 1 to 7, this is limited by the largest distance allowed to the eye being probed, in order to get a dependable result. Furthermore, data from a range of points is used for fitting the localization curve, wherein the data for first ten points are collected for U+ voltage and U- voltage, respectively. Additionally, the first five Z-values are calculated from the given data. This has been explained further in detail in conjunction with FIG. 13C. Subsequently, values as provided in table of FIG. 13D were observed. Furthermore, by removing the speed, the magnetization and the dependence of the elongated magnetic probe on the first induced voltage value, the second induced voltage value, the third induced voltage value and the fourth induced voltage value, and dividing them point by point with the common induced voltage, the position of the elongated magnetic probe is obtained. Additionally, a suitable linear combination may be created with the help of the measurements. This has been explained further in detail in conjunction with FIG. 5

EXAMPLE 2: An example of how to determine above mentioned look-up table by moving the elongated magnetic probe back and forth

In the present disclosure, the first induced voltage used for indicating the speed of the elongated magnetic probe is induced in the measurement coil by vibrating the elongated magnetic probe using a vibrator. Herein, the vibrator vibrates the elongated magnetic probe at a frequency of 875 hertz (Hz), using a commercial piezoelectric device at its mechanical resonance frequency. Typically, the amplitude of the vibration of the vibrator is small. Notably, the vibrator is driven by setting a signal generator to 0.2 volt (V) and 0.3 V, respectively. Moreover, the common induced voltage is the sum of the induced voltages of the measurement coil. Additionally, the sum of the voltages of the first section and the second section may be measured by measuring the induced voltage of the first section and the second section separately.

Optionally, the common induced voltage is measured over all sections of the measurement coil. Herein, at least the first section and the second section of the measurement coil are connected with a first signal amplifier and a second signal amplifier. Furthermore, the first signal amplifier and the second signal amplifier comprise differential inputs that may be modified as per requirement. In an exemplary implementation, the first signal amplifier is connected between the first section and the third section, and the second signal amplifier is connected between the third section and the fourth section. Furthermore, the vibrator produces signals from the first signal amplifier and the second signal amplifier below 1 volt (V), which is low enough to avoid saturation. Typically, 500 root mean square of the measured voltage in millivolt (mVrms) is the output for the driver coil, wherein standard voltage of the driver coil is 0.2 V. Furthermore, 800 mVrms is the output for the driver coil whose standard voltage is 0.3V. Additionally, the inputs of the first signal amplifier and the second signal amplifier are connected to the plurality of coupling leads of the measurement coil, wherein the measurement coil corresponds to about 1 millivolt (mV) and 1.6 mV respectively. In addition, the outputs of the first signal amplifier and second signal amplifier respectively are connected to inputs of a digital oscilloscope for measuring the speed of the elongated magnetic probe. Particularly, induced voltages are obtained from the four sections of the measurement coil. Herein, induced voltage obtained between the first coupling lead and the second coupling lead is referred to as 'U12', voltage obtained between the second coupling lead and the coupling lead is referred to as 'U23', voltage obtained between the third coupling lead and the coupling lead is referred to as 'U34', and voltage obtained between the fourth coupling lead and the fifth coupling lead is referred to as 'U45'. Herein, the measurements are taken over a train of 30 magnetization cycles, by manually triggering the signal generator using the 0.2 V driver coil or the 0.3 V driver coil at 875 hertz (Hz), wherein the signal generator is connected to the driver coil through a third signal amplifier. Consequently, the digital oscilloscope measures the root mean square value of the output from the first signal amplifier and the second signal amplifier and saves it digitally into a spreadsheet.

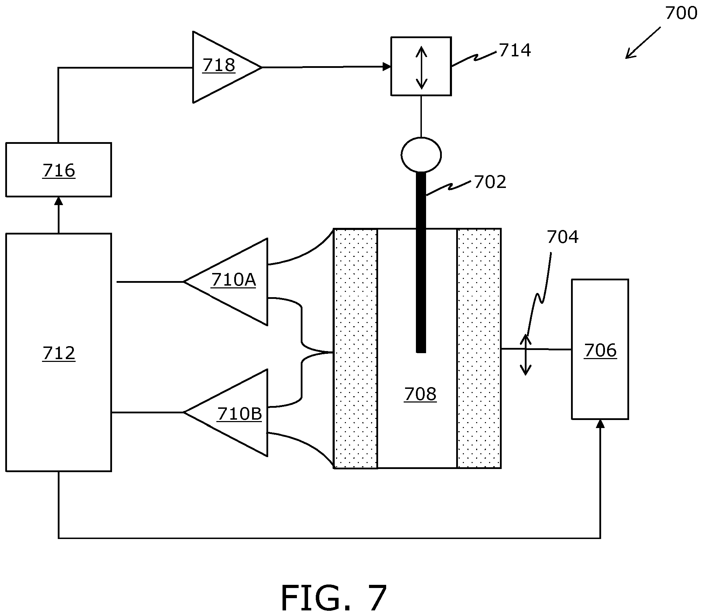

Herein, the spreadsheet comprises visual basic code for receiving and displaying the measurements as a function of the position of the elongated magnetic probe. Additionally, the position of the elongated magnetic probe is determined by integrating the speed over time. Optionally, the position of the elongated magnetic probe is controlled by a robotic system. Herein, the robotic system comprises a linear screw driven by a step motor, allowing the elongated magnetic probe move linearly with a high accuracy. Beneficially, slow movement of the measurement coil does not induce a voltage in the signal coil. Furthermore, the robotic system steps the position between measurements. Herein, the position of the elongated magnetic probe is referred to as 'Z'. Additionally, the mechanical end position is starting position, Z = 0. Furthermore, the robotic system is also triggered to step 1 millimetre (mm) for the next point to be measured. This has been explained further in detail in conjunction with FIG. 7.

In an implementation of the present disclosure, the locator values were observed to be linear in a range of Z = 1 to 5 millimetres (mm). (Z being distance of probe moving in respect to coils). Notably, the trend line is fit using the spreadsheet to the points in the range of Z = 1 millimetre to 5 millimetres, thereby providing a close approximation. Furthermore, the resulting trend line of the locator values, has the form of general equation of the straight line, $y = mx + c$. Additionally, the trend line represents the position of the elongated magnetic probe in the range of Z = 1 millimetre to 5 millimetres for doing measurement in the apparatus. Herein, variables 'x' and 'y' are chosen for further calculations. Subsequently, variable x is made equivalent to Z and variable y is made equivalent to 1000 times of the function of L(Z), in order to fit with the coordinates in the present disclosure. Therefore, the equation of the trend line is given by

$$1000L = -mZ + c$$

Herein, the value of Z is calculated by inversion of the previous equation

$$Z = -\left(\frac{1000}{m}\right)L + \frac{c}{m}$$

Herein, $K = \frac{-1000}{m}$ , wherein K is a constant. Furthermore, $c/m$ is a value of Z for L=0. Subsequently, $c/m = Z_o$.

Hence, $V = K\left(\frac{\mathrm{dL}}{dt}\right)$ , wherein V is a variable. Consequently,

$$Z = -Vt + Z_o$$

Thus based on the example and example test we are able to determine from the locator values velocity profile of the elongated magnetic probe. The velocity profile can be used to determine a physiological parameter. I.e the speed of the elongated magnetic probe may be used to determine the physiological parameters. Notably, the first induced voltage is proportional to the speed of the elongated magnetic probe

DETAILED DESCRIPTION OF THE DRAWINGS

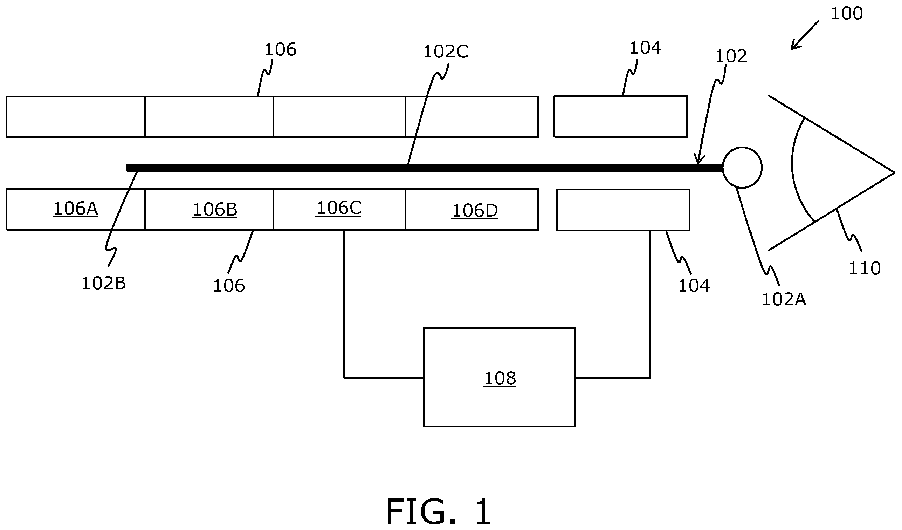

Referring to FIG. 1, there is shown a block diagram of an apparatus **100** for determining physiological parameter, in accordance with an implementation of the present disclosure. The apparatus **100** comprises an elongated magnetic probe **102,** a driver coil **104,** a measurement coil **106** and a controller **108.** The elongated magnetic probe has a first end **102A,** a second end **102B** opposite to the first end **102A** and a middle section **102C** between the first end **102A** and the second end **102B.** Furthermore, the driver coil **104** is arranged partially to surround the elongated magnetic probe **102.** Additionally, a measurement coil **106** comprises at least a first section **106A** and a second section **106B,** and the measurement coil **106** is arranged partially to surround the elongated magnetic probe **102.** In the figure the measurement coil comprises 4 sections, the first section **106A** , the second section **106B,** a third section **106C** and a fourth section **106D.** Sections are connected with each other's in series. Herein, a controller **108** is communicably coupled with the driver coil **104** and the measurement coil **106.** Herein, the controller **108** selectively energizes the driver coil **104** to create a magnetic force to initiate movement of the elongated magnetic probe **102** towards an eye **110.** Figure 1 illustrates probe at position Z=0. As an example a first induced voltage can be measured over the first section 106A. Common induced voltage can be measured over the first section 106A and the second section 106B. Other example is to measure the first induced voltage over the second section 106B and the common induced voltage for example over all four sections. Indeed the term "first section" can refer to any section. The common induced voltage is measured from at least some section(s) which are also different from the first section.

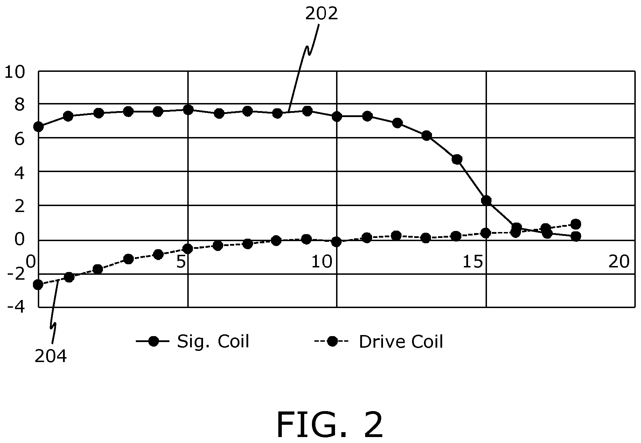

Referring to FIG. 2, there is shown a graph to depict a force on elongate magnetic probe **102** induced per ampere of current in signal or drive coil versus position in millimeters, in accordance with an implementation of the present disclosure. Herein, horizontal axis represents the magnetic force acting on the elongated magnetic probe **102** induced for 1 ampere (A) of electric current in the measurement coil **106** and the driver coil **104.** The vertical axis represents the position of the elongated magnetic probe **102** in millimeters. Furthermore, the line **202** illustrates the magnetic force acting on the elongated magnetic probe **102** by an electric current in the measurement coil **106.** Furthermore, the line **204** illustrates the magnetic force acting on the elongated magnetic probe **102** by an electric current in the driver coil **104.** The upper curve **202** corresponds to the single signal coil used in present instruments, the lower one **204** to the drive coil. The curves for signal voltages induced by a probe moving at constant velocity in the coils look the same.

Referring to FIG. 3, there is shown a graph **302** to determine the common induced voltage of the measurement coil **106,** in accordance with an implementation of the present disclosure. Herein, the common induced voltage is a function of time. At point **304,** the common induced voltage increases as the driver coil **104** begins to accelerate the elongated magnetic probe **102** (starting from position Z=0 mm). Subsequently, the elongated magnetic probe **102** hits the surface of the eye **110** at point **306** (at said when the position is between Z=4 to 8 mm)and decelerates for about 1 millisecond (ms) to zero after from 20 ms up to 35 ms and then bounces back, depending on the exact velocity and distance from the surface of the eye **110.** Herein, the trajectory of the speed of the elongated magnetic probe **102** during contact with the surface of the eye **110** is mainly dependent on the speed of the elongated magnetic probe **102** and, via a pushback force, on intra-ocular pressure. Furthermore, the main parameter for dependence on the intra-ocular pressure is given by the slope of deceleration of the speed of the elongated magnetic probe **102,** wherein deceleration of the speed of the elongated magnetic probe **102** is equal to the magnetic force between the elongated magnetic probe **102** and the surface of the eye **110.** The figure illustrates measurement over a single section of a measurement coil. The drive coil is active at Z<1. The probe hits the eye approximately at Z=5-7 mm were the drive force is close to zero.

Fig 4 illustrates three locator signals using four differential amplifiers to measure the voltages across each of the signal coil sections. The curves are made by taking signals from adjacent coils, a first section and a second section, the second section and a third section, and the third section and a fourth section, subtracting them pairwise, and dividing them with the sum U+ over all of them (common induced voltage). The curves calculated as described are all independent of probe velocity and magnetization. Selecting between the curves one can localize the probe over a large range. One can also form a suitable linear combination to optimize the sensitivity for a selected region. This setup requires four amplifier/detectors synchronized to sample each point simultaneously by all four channels. Benefit of measuring more than one first induced voltage values is increase of accuracy.

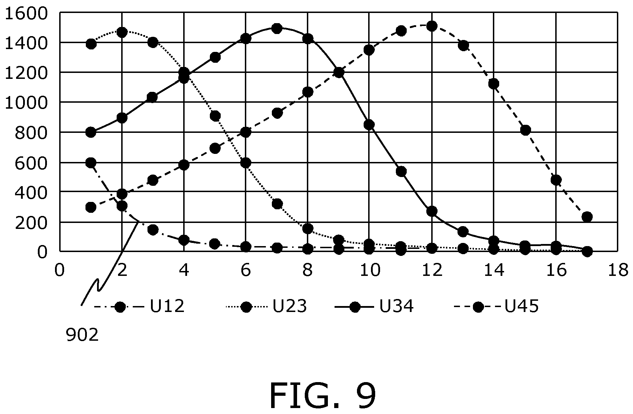

Referring to FIG. 5, there is shown a graph to determine the position of the elongated magnetic probe **102,** in accordance with an implementation of the present disclosure. Herein, the dependence of velocity and magnetization of the elongated magnetic probe **102** are removed by measuring the induced voltage U13 in a half section of the measurement coil (=U12+U23 in Fig.9) and the one U35 across the other half (=U34+U45). A signal ratio S is calculated as the difference of U13-U34 divided by the common voltage Ucom across the whole coil, which in this case is equal to the sum U13+U34. This signal ratio S represents the position of the elongated magnetic probe 102, and is independent of probe magnetization and velocity. Herein, the horizontal axis represents the position of the elongated magnetic probe **102** in millimeters. Herein, the vertical axis represents the signal ratio S, which is a function of said probe position. Using two signal points, S1 and S2 at times t1 and t2, the curve gives us the corresponding z1 and z2. Using such signal points, the mean velocity of the probe is calculated as $v_{cal}$= (z1-z2)/(t1-t2). Thus we can determine the probe position directly and the probe velocity indirectly, independently of probe magnetization

Indeed we can calibrate influence of magnetization and use it to derive probe position and velocity without knowing the magnetization factor of the probe.

Referring to FIG. 6, there is shown an assembly of the measurement coil **602,** in accordance with an implementation of the present disclosure. Herein, the measurement coil **602** is split into a first section denoted by **602A,** a second section denoted by **602B,** a third section denoted by **602C** and a fourth section denoted by **602D.** Furthermore, a first coupling lead **604A** is connected to the leftmost end **J1** of the first section **602A,** a second coupling lead **604B** is connected to the junction **J2** of the first section **602A** and the second section **602B,** a third coupling lead **604C** is connected to the junction **J3** of the second section **602B** and the third section **602C,** a fourth coupling lead **604D** is connected to the junction **J4** of the third section **602C** and the fourth section **602D,** and a fifth coupling lead **604E** is connected to the rightmost end **J5** of the fourth section **602D.** Additionally, the length of the measurement coil **608** may be 20 millimeters (mm). Moreover, the elongated magnetic probe **610** moves linearly with an accuracy **612** of 1 mm. Herein, Z = 0 is the starting position of the elongated magnetic probe **610.** In general about notations used in the description following examples are provided: U12 refers to induced voltage between junctions J1 and J2. U16 refers to induced voltage between junctions J1 and J5 (i.e. voltages over all sections **602A, 602B, 602C** and **602D).** U34 refers induced voltage between junctions J3 and J4. U24 refers to induced voltage between junctions J2 and J4 i.e. over the second section **602B** and third section **603C.**

Referring to FIG. 7, there is shown a schematic illustration of a system **700** for a robotic system to determine the speed and position of the elongated magnetic probe **702,** in accordance with an embodiment of the present disclosure. Herein, the robotic system comprises a linear screw **704** driven by a step motor **706,** allowing the elongated magnetic probe **702** to move linearly inside the measurement coil **708** with better accuracy. Furthermore, a first signal amplifier **710A** and a second signal amplifier **710B** are used to amplify the induced voltage of the measurement coil **708.** Subsequently, the first signal amplifier **710A** and the second signal amplifier **710B** are electrically coupled with an oscilloscope **712.** Additionally, the elongated magnetic probe **702** is vibrated by a vibrator **714** at a frequency of 875 hertz (Hz) to determine the speed of the elongated magnetic probe **702.** Herein, the vibrator **714** is driven by a signal generator **716** via a third amplifier **718.**

Referring to FIG. 8, there is shown an electric circuit **800** to add the induced voltages, in accordance with an implementation of the present disclosure. Herein, a plurality of resistors are denoted by **R1, R2, R3** and **R4.** Furthermore, a first voltage input connected between the first section and the second section of the measurement coil **708** is denoted by **V1,** a second voltage input connected at the end of the fourth section of the measurement coil **708** is denoted by **V2,** a third voltage input connected between the third section and the fourth section of the measurement coil **708** is denoted by **V3.** Firstly, the first voltage input is negative form of the second induced voltage value, i.e., V1= -U2. Secondly, the second voltage input is the addition of the third induced voltage value and the second induced voltage value, i.e., V2 = U3 + U4. Thirdly, the third input is equivalent to the third induced voltage, V3 = U3. Finally, an output voltage denoted by $V_{out}$ is obtained, wherein the output voltage is the addition of the first input, the second input and the third input

$$V_{out} = V1+V2+V3 = -U2+U3+U4-U3 = U4-U2$$

Furthermore, the output voltage may also be calculated with respect to the plurality of resistors,

$$V1 = \left(V1 \cdot \frac{R1}{R1 + R2} + V2 \cdot \frac{R1}{R1 + R2}\right)\left(1 + \frac{R4}{R3}\right) - V3 \cdot \frac{R4}{R3}$$

Referring to FIG. 9, there is shown induced signal voltages in the measurement coil **602,** in accordance with an implementation of the present disclosure. Herein, the vertical axis represents twice the approximated root mean square value in millivolt. Furthermore, the induced voltage obtained between the first coupling lead **604A** and the second coupling lead **604B** is referred to as **U12,** voltage obtained between the second coupling lead **604B** and the third coupling lead **604C** is referred to as **U23,** voltage obtained between the third coupling lead **604C** and the fourth coupling lead **604D** is referred to as **U34,** and voltage obtained between the fourth coupling lead **604D** and the fifth coupling lead **604E** is referred to as **U45.**

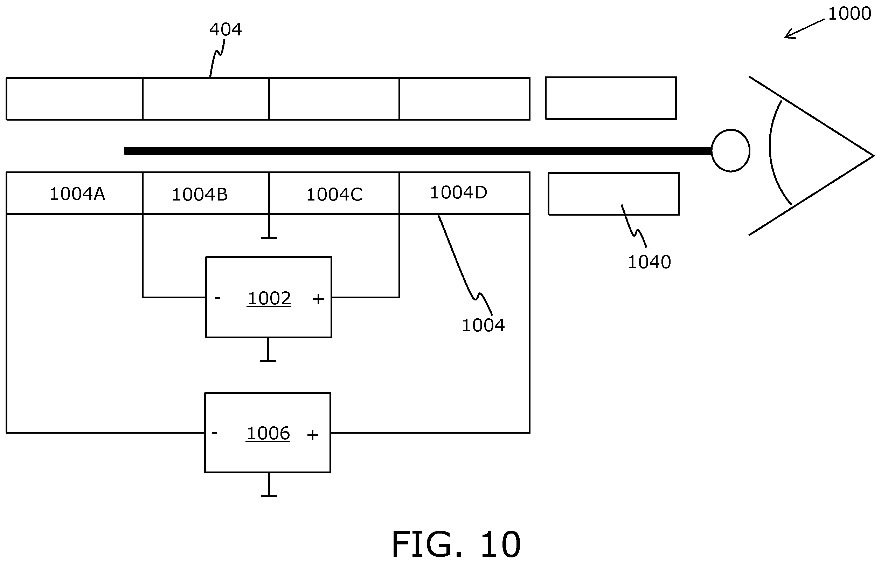

Referring to FIG. 10, there is shown a block diagram for a system **1000** for implementation of the apparatus, in accordance with an exemplary implementation of the present disclosure. Herein, a first amplifier **1002** is electrically coupled with the second section **1004B** and the third section **1004C** of the measurement coil **1004** to measure induced voltages of the second section and the third section to be used as the first induced voltage values. Furthermore, a second amplifier **1006** is electrically coupled with the first section 1004A and the fourth section **1004D** of the measurement coil **1004** to determine the common induced voltage. Probe is in position Z=0 in the figure. Probe can be moved with driver coil 1040.

Referring to FIGs. 11A and 11B, there are shown graphs to realize an optimal connection for the first amplifier **1002** of figure 10 and the second amplifier **1006** of figure 10, in accordance with an implementation of the present disclosure. As shown previously, the first amplifier **1002** is electrically coupled with the second section and the third section of the measurement coil **1004** as shown by the graph of figure 10. Furthermore, the second amplifier **1006** is electrically coupled with the first section and the fourth section of the measurement coil **1004.** Herein, for a vibrator of 0.2 volts (V), the common induced voltage denoted by **U15** is plotted alongside the induced voltage from the second and the fourth section denoted by **U24.** Furthermore, **U24** is divided with **U15** to find the optimal connection of the first amplifier **1002** and the second amplifier **1006.** Additionally, in FIG. 11B, for a vibrator of 0.3 V, the common induced voltage denoted by **U15** is plotted alongside the induced voltage from the second and the fourth section denoted by **U24.** Furthermore, **U24** is divided with **U15** to find the optimal connection of the first amplifier **1002** and the second amplifier **1006.**

FIG 12A is an illustration of measured first voltage values U13 and U35.

FIG 12B represents signal voltages, added to represent the total of the signal coil sections (U+), and their difference (U-). Note that U+ is equal to U15: The notation is different just to show that it has been measured by adding two or more measured signals: This is convenient when using them for several purposes, e.g. to form the difference U. A minimal system that would operate like this: The signal coil would have two sections, each with a differential amplifier to generate the signals shown in Fig.12A. Instead of forming a U- and U+ signal (FIG 12B), and their quotient (the Locator) one could use the zero-crossing point at 4mm. Provided that the time for this occurrence has been measured we can calculate the mean speed to go from 0 to 4mm.

Referring to FIGs. 13A to 13D, there is shown graph to measure the locator values by the addition of plurality of signal amplifiers, in accordance with an implementation of the present disclosure. In FIG. 13A, the line *'Series 1'* denotes U13, wherein U13 is the induced voltage in the measurement coil **602** between the first coupling lead **604A** and third coupling lead **604C.** Similarly, the line *'Series 2'* denotes U35, wherein U35 is the induced voltage in the measurement coil

602 between the third coupling lead 604C and the fifth coupling lead 604E. Furthermore, the line 'Series 3' is U+, i.e., summation of the output signals from the first signal amplifier and the second signal amplifier. Furthermore, U- is the difference between the output from the first signal amplifier and the second signal amplifier. FIG. 13B provides locator values with respect to U+ and U-. Notably, the locator signal is generated by dividing U- by U+. Furthermore, FIG 13A and 13B provide data while making long sweep of Z-values. Similarly, FIG. 13C provides data from the range of induced voltage values used for fitting trend line. FIG. 13D provides locator values with respect to U+ and U-.

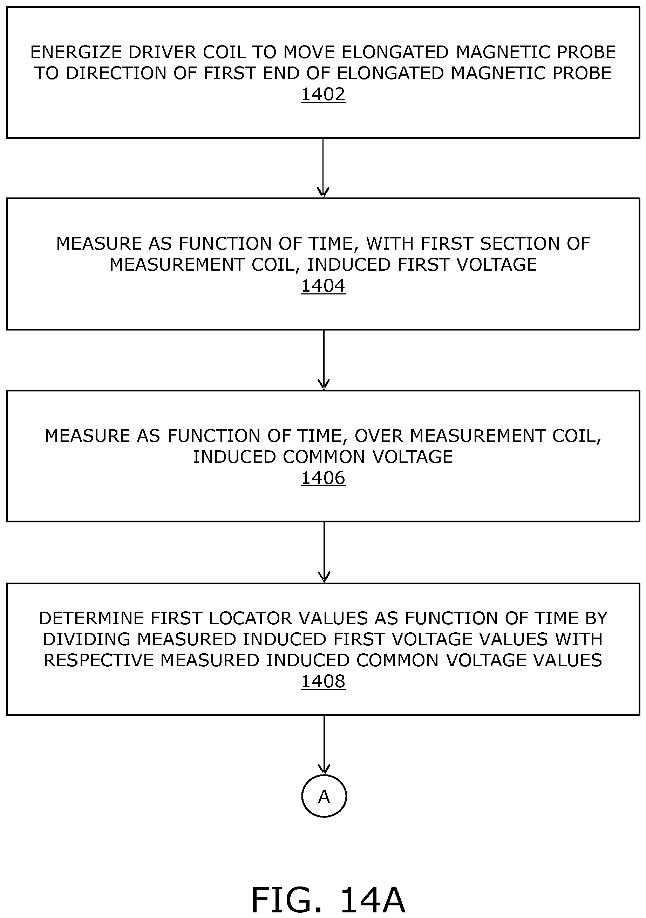

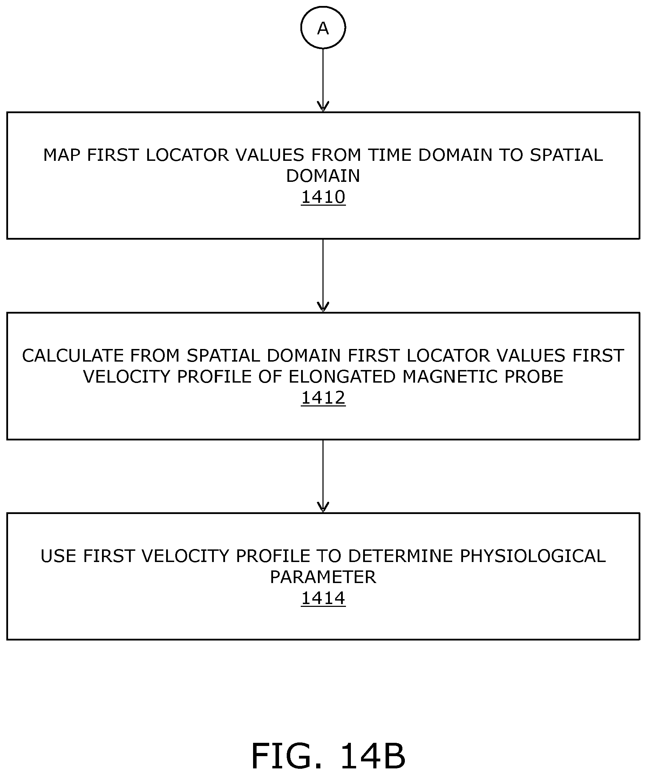

Referring to FIGs. 14A and 14B collectively illustrate a flowchart depicting steps of a method for determining a physiological parameter, in accordance with an embodiment of the present disclosure. At a step 1402, a driver coil is energized to move an elongated magnetic probe to a direction of first end of the elongated magnetic probe. At a step 1404, an induced first voltage is measured as a function of time, with a first section of a measurement coil. At a step 1406, an induced common voltage is measured as a function of time, over the measurement coil. At a step 1408, a first locator value is determined as a function of time by dividing the measured induced first voltage values with respective measured induced common voltage values. At a step 1410, the first locator values are mapped from time domain to a spatial domain. At a step 1412, the spatial domain first locator values are used for calculating a first velocity profile of the elongated magnetic probe. At a step 1414, the first velocity profile is used to determine the physiological parameter.

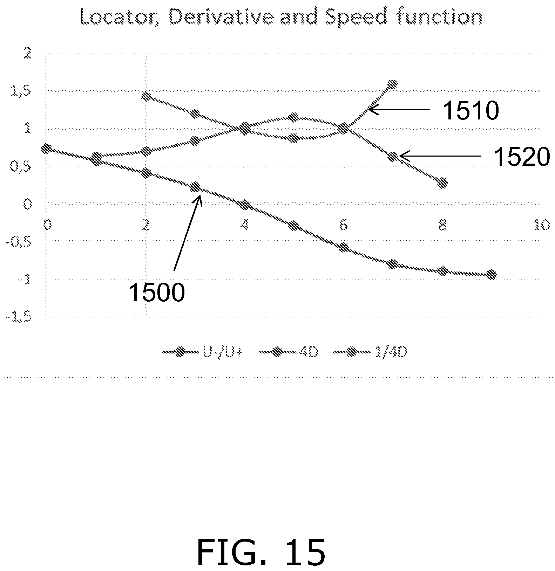

FIG 15 is illustration of an measurement and calculated values as function fo probe movement (Z-axis in mm 0 to 10 mm). The locator 1500, is formed by taking the ratio of the U- and U+ signals shown in Fig 12B. The derivative 1510 of the locator 1500 curve is formed dividing the increment of the points by the step length (1 mm). (The derivate curve 1510 is multiplied by -4, to make the FIG 15 more easy to read). The inverse of the derivative curve 1520, is a first velocity profile as function of distance, in mm.

Modifications to embodiments of the present disclosure described in the foregoing are possible without departing from the scope of the present disclosure as defined by the accompanying claims. Expressions such as "including", "comprising", "incorporating", "have", "is" used to describe and claim the present disclosure are intended to be construed in a non-exclusive manner, namely allowing for items, components or elements not explicitly described also to be present. Reference to the singular is also to be construed to relate to the plural.

## Claims

1.    An apparatus (100) for determining physiological parameter of an eye, the apparatus comprising;

    - an elongated magnetic probe (102, 610, 702), having a first end (102A), a second end (102B) opposite to the first end and a middle section (102C) between the first end and the second end;
    - a driver coil (104) arranged partially to surround the elongated magnetic probe
    - a measurement coil (106, 602, 708, 1004) comprising at least a first section (106A) and a second section (106B), and the measurement coil arranged partially to surround the elongated magnetic probe and
    - a controller (108) configured to

        - selectively energize the driver coil to create a magnetic force to initiate movement of the elongated magnetic probe to a direction of the first end, wherein the elongated magnetic probe is directed to hit a surface of the eye and bounce back thereof;
        - measure first induced voltage values over the first section and common induced voltage values over at least one of: the first section and the second section, or over the second section, as a function of time during a time of the movement of the elongated magnetic probe;
        - determine locator values as a function of time by dividing the first induced voltage values with the common induced voltage values;
        - map the locator values as a function of time from the time domain to a spatial domain and
        - calculate from the spatial domain locator values, a first velocity profile of the elongated magnetic probe, and use the calculated first velocity profile of the elongated magnetic probe to determine the physiological parameter of the eye.

2.    An apparatus (100) according to claim 1, wherein the measurement coil (106, 602, 708, 1004) is used during first period of time as the driver coil (104) and during a second period of time, which the second period of time is after the first period of time, as the second section of the measurement coil.

3.    An apparatus (100) according to any of the preceding claims wherein, when in use, the first section of the measurement coil (106, 602, 708, 1004) surrounds a first section of the elongated magnetic probe (102, 610, 702) and the second section of the measurement coil surrounds a second section of the elongated magnetic probe, wherein the first section of the elongated magnetic probe is different from the second section of the elongated magnetic probe, when the elongated magnetic probe is in its first spatial position.

4. An apparatus (100) according to claim 3, wherein, when in use, the first section of the measurement coil (106, 602, 708, 1004) does not surround the first section of the elongated magnetic probe (102, 610, 702) and the second section of the measurement coil surrounds the second section of the elongated magnetic probe, when the elongated magnetic probe is in its second spatial position, which the second spatial position is different from the first spatial position.

5. An apparatus (100) according to any of the preceding claims, wherein the time domain to spatial domain is mapped by at least one of: pre-determined transfer function or a look up table.

6. An apparatus (100) according to any of the preceding claims, wherein the first section and the second section of the measurement coil (106, 602, 708, 1004) are connected in series.

7. An apparatus (100) according to any of the preceding claims, wherein the measurement coil (106, 602, 708, 1004) comprises a third section, the third section connected in series with the first and the second section of the measurement coil and the third section is arranged to surround a third section of the elongated magnetic probe (102, 610, 702).

8. An apparatus (100) according to any of the preceding claims, wherein the common induced voltage is measured over all sections of the measurement coil (106, 602, 708, 1004).

9. An apparatus (100) according to any of the preceding claims, wherein controller (108) is further configured to

- measure a second induced voltage values from a section other than the first section, as a function of time during a time of the movement of the elongated magnetic probe (102, 610, 702);
- determine a second locator values as function of time by dividing the second induced voltage values with the common induced voltage values;
- map the second locator function from a time domain to a spatial domain;
- calculate from the spatial domain second locator values a second velocity profile of the elongated magnetic probe and
- use the calculated second velocity profile the elongated magnetic probe to update the physiological parameter of the eye.

10. An apparatus (100) according to any of the preceding claims, wherein the first velocity profile comprises at least one of: a velocity in the spatial domain, a velocity in the time domain.

11. An apparatus (100) according to any of the preceding claims 9-10, wherein the second velocity profile comprises at least one of: a velocity in the spatial domain, a velocity in the time domain.

12. An apparatus (100) according to any of the preceding claims, wherein the apparatus is a tonometer.

13. A method for determining a physiological parameter of an eye, the method using a controller to carry out the method steps comprising:

- energizing a driver coil (104) to move elongated magnetic probe (102, 610, 702) to a direction of a first end (102A) of the elongated magnetic probe, wherein the elongated magnetic probe is directed to hit a surface of the eye and bounce back thereof;
- measuring as a function of time, over a first section of a measurement coil (106, 602, 708, 1004), an induced first voltage;
- measuring as a function of time, over at least one of: a first section and a second section or over the second section of the measurement coil, an induced common voltage;
- determining a first locator value as a function of time by dividing the measured induced first voltage values with respective measured induced common voltage values;
- mapping the first locator values from time domain to a spatial domain;
- calculating from the spatial domain first locator values a first velocity profile of the elongated magnetic probe and
- using the first velocity profile to determine the physiological parameter of the eye.

14. A method according to claim 13, wherein the measurements are carried out during the movement of the elongated magnetic probe (102, 610, 702) to obtain induced voltage values as function of time.

15. A method according to any of the claims 13-14, wherein the determination of the physiological parameter of the eye is carried out at least by one of: analysing acceleration of the elongated magnetic probe (102, 610, 702) during its impact to the surface of the patient body, change of velocity before the impact and after the impact, amount of penetration of the elongated magnetic probe to the surface of the body.

16. A method according to any of the claims 13 to 15, wherein the physiological parameter value of the eye is updated by

- measuring as a function of time, with a section of a measurement coil (106, 602, 708, 1004) different from the first section, a second induced first voltage;
- determining a second locator values as function of time by dividing the second induced voltage values with the common induced voltage values;
- mapping the second locator function from a time domain to a spatial domain;
- calculating from the spatial domain second locator values, a second velocity profile of the elongated magnetic probe (102, 610, 702) as function of location;
- using the calculated second velocity profile for updating the physiological parameter of the eye.

17. A method according to any of the claims 13-16, wherein the first velocity profile comprises at least one of: a velocity in the spatial domain, a velocity in the time domain.

18. A method according to any of the claims 16-17, wherein the second velocity profile comprises at least one of: the velocity in the spatial domain, the velocity in the time domain.

**Patentansprüche**

1. Einrichtung (100) zum Bestimmen eines physiologischen Parameters eines Auges, die Einrichtung umfassend;

- eine längliche Magnetsonde (102, 610, 702), die ein erstes Ende (102A), ein zweites Ende (102B) gegenüber dem ersten Ende und einen Mittelabschnitt (102C) zwischen dem ersten Ende und dem zweiten Ende aufweist;
- eine Treiberspule (104), die angeordnet ist, um die längliche Magnetsonde teilweise zu umgeben
- eine Messspule (106, 602, 708, 1004), umfassend mindestens einen ersten Abschnitt (106A) und einen zweiten Abschnitt (106B), und wobei die Messspule angeordnet ist, um die längliche Magnetsonde teilweise zu umgeben, und
- eine Steuervorrichtung (108), die konfiguriert ist zum
- selektiven Erregen der Treiberspule, um eine magnetische Kraft zu erzeugen, um eine Bewegung der länglichen magnetischen Sonde in eine Richtung des ersten Endes zu initiieren, wobei die längliche magnetische Sonde ausgerichtet ist, um eine Oberfläche des Auges zu treffen und davon zurückzuprallen;
- Messen erster induzierter Spannungswerte über dem ersten Abschnitt und gemeinsamer induzierter Spannungswerte über mindestens einem von: dem ersten Abschnitt und dem zweiten Abschnitt, oder über dem zweiten Abschnitt, in Abhängigkeit von der Zeit während einer Zeit der Bewegung der länglichen Magnetsonde;
- Bestimmen von Lokalisiererwerten in Abhängigkeit von der Zeit durch Dividieren der ersten induzierten Spannungswerte durch die gemeinsamen induzierten Spannungswerte;
- Abbilden der Lokalisiererwerte in Abhängigkeit von der Zeit aus dem Zeitbereich in einen räumlichen Bereich, und
- Berechnen aus den Lokalisiererwerten des räumlichen Bereichs eines ersten Geschwindigkeitsprofils der länglichen Magnetsonde und Verwenden des berechneten ersten Geschwindigkeitsprofils der länglichen Magnetsonde, um den physiologischen Parameter des Auges zu bestimmen.

2. Einrichtung (100) nach Anspruch 1, wobei die Messspule (106, 602, 708, 1004) während einer ersten Zeitperiode als die Treiberspule (104) und während einer zweiten Zeitperiode, wobei die zweite Zeitperiode nach der ersten Zeitperiode liegt, als der zweite Abschnitt der Messspule verwendet wird.

3. Einrichtung (100) nach einem der vorstehenden Ansprüche, wobei, wenn in Verwendung, der erste Abschnitt der Messspule (106, 602, 708, 1004) einen ersten Abschnitt der länglichen Magnetsonde (102, 610, 702) umgibt und der zweite Abschnitt der Messspule einen zweiten Abschnitt der länglichen Magnetsonde umgibt, wobei sich der erste Abschnitt der länglichen Magnetsonde von dem zweiten Abschnitt der länglichen Magnetsonde unterscheidet, wenn sich die längliche Magnetsonde in ihrer ersten räumlichen Position befindet.

4. Einrichtung (100) nach Anspruch 3, wobei, wenn in Verwendung, der erste Abschnitt der Messspule (106, 602, 708, 1004) den ersten Abschnitt der länglichen Magnetsonde (102, 610, 702) nicht umgibt und der zweite Abschnitt der Messspule den zweiten Abschnitt der länglichen Magnetsonde umgibt, wenn sich die längliche Magnetsonde in ihrer zweiten räumlichen Position befindet, wobei die zweite räumliche Position von der ersten räumlichen Position verschieden ist.

5. Einrichtung (100) nach einem der vorstehenden Ansprüche, wobei der Zeitbereich zu dem räumlichen Bereich durch mindestens eines der Folgenden abgebildet wird: zuvor bestimmte Übertragungsfunktion oder eine Nachschlagetabelle.

6. Einrichtung (100) nach einem der vorstehenden Ansprüche, wobei der erste Abschnitt und der zweite Abschnitt der Messspule (106, 602, 708, 1004) in Reihe geschaltet sind.

7. Einrichtung (100) nach einem der vorstehenden Ansprüche, wobei die Messspule (106, 602, 708, 1004) einen dritten Abschnitt umfasst, wobei der dritte Abschnitt mit dem ersten und dem zweiten Abschnitt der Messspule in Reihe geschaltet ist und der dritte Abschnitt angeordnet ist, um einen dritten Abschnitt der länglichen Magnetsonde (102, 610, 702) zu umgeben.

8. Einrichtung (100) nach einem der vorstehenden Ansprüche, wobei die gemeinsame induzierte Spannung über alle Abschnitte der Messspule (106, 602, 708, 1004) gemessen wird.

9. Einrichtung (100) nach einem der vorstehenden Ansprüche, wobei die Steuervorrichtung (108) ferner konfiguriert ist zum

- Messen zweiter induzierter Spannungswerte von einem anderen Abschnitt als dem ersten Abschnitt, in Abhängigkeit von der Zeit während einer Zeit der Bewegung der länglichen Magnetsonde (102, 610, 702);
- Bestimmen zweiter Lokalisiererwerte in Abhängigkeit von der Zeit durch Dividieren der zweiten induzierten Spannungswerte durch die gemeinsamen induzierten Spannungswerte;
- Abbilden der zweiten Lokalisiererfunktion von einem Zeitbereich auf einen räumlichen Bereich;
- Berechnen aus dem räumlichen Bereich von zweiten Lokalisiererwerten eines zweiten Geschwindigkeitsprofils der länglichen Magnetsonde, und
- Verwenden des berechneten zweiten Geschwindigkeitsprofils der länglichen Magnetsonde, um den physiologischen Parameter des Auges zu aktualisieren.

10. Einrichtung (100) nach einem der vorstehenden Ansprüche, wobei das erste Geschwindigkeitsprofil mindestens eines umfasst von: einer Geschwindigkeit in dem räumlichen Bereich, einer Geschwindigkeit in dem Zeitbereich.

11. Einrichtung (100) nach einem der vorstehenden Ansprüche 9 bis 10, wobei das zweite Geschwindigkeitsprofil mindestens eines umfasst von: einer Geschwindigkeit in dem räumlichen Bereich, einer Geschwindigkeit in dem Zeitbereich.

12. Einrichtung (100) nach einem der vorstehenden Ansprüche, wobei die Einrichtung ein Tonometer ist.

13. Verfahren zum Bestimmen eines physiologischen Parameters eines Auges, wobei das Verfahren eine Steuervorrichtung verwendet, um die Verfahrensschritte vorzunehmen, umfassend:

- Erregen einer Treiberspule (104), um eine längliche Magnetsonde (102, 610, 702) in eine Richtung eines ersten Endes (102A) der länglichen Magnetsonde zu bewegen, wobei die längliche Magnetsonde ausgerichtet ist, um eine Oberfläche des Auges zu treffen und davon zurückzuprallen;
- Messen in Abhängigkeit von der Zeit, über einen ersten Abschnitt einer Messspule (106, 602, 708, 1004), einer induzierten ersten Spannung;
- Messen in Abhängigkeit von der Zeit, über mindestens einem von: einem ersten Abschnitt und einem zweiten Abschnitt oder über dem zweiten Abschnitt der Messspule, einer induzierten gemeinsamen Spannung;
- Bestimmen eines ersten Lokalisiererwerts in Abhängigkeit von der Zeit durch Dividieren der gemessenen induzierten ersten Spannungswerte durch entsprechende gemessene induzierte gemeinsame Spannungswerte;
- Abbilden der ersten Lokalisiererwerte von dem Zeitbereich auf einen räumlichen Bereich;
- Berechnen aus den ersten Lokalisiererwerten des räumlichen Bereichs eines ersten Geschwindigkeitsprofils der länglichen Magnetsonde und
- Verwenden des ersten Geschwindigkeitsprofils, um die physiologischen Parameters des Auges zu bestimmen.

14. Verfahren nach Anspruch 13, wobei die Messungen während der Bewegung der länglichen Magnetsonde (102, 610, 702) vorgenommen werden, um induzierte Spannungswerte in Abhängigkeit von der Zeit zu erhalten.

15. Verfahren nach einem der Ansprüche 13 bis 14, wobei die Bestimmung des physiologischen Parameters des Auges mindestens durch eines vorgenommen wird von: Analysieren der Beschleunigung der länglichen magnetischen Sonde (102, 610, 702) während ihres Aufpralls auf die Oberfläche des Patientenkörpers, Ändern der Geschwindigkeit vor dem Aufprall und nach dem Aufprall, Ausmaß eines Eindringens der länglichen magnetischen Sonde in die Oberfläche des Körpers.

16. Verfahren nach einem der Ansprüche 13 bis 15, wobei der Wert des physiologischen Parameters des Auges aktualisiert wird durch

- Messen in Abhängigkeit von der Zeit, mit einem Abschnitt einer Messspule (106, 602, 708, 1004), der sich von dem ersten Abschnitt unterscheidet, einer zweiten induzierten ersten Spannung;
- Bestimmen zweiter Lokalisiererwerte in Abhängigkeit von der Zeit durch Dividieren der zweiten induzierten Spannungswerte durch die gemeinsamen induzierten Spannungswerte;
- Abbilden der zweiten Lokalisiererfunktion von einem Zeitbereich auf einen räumlich Bereich;
- Berechnen aus zweiten Lokalisiererwerten des zweiten Bereichs eines zweiten Geschwindigkeitsprofils der länglichen Magnetsonde (102, 610, 702) in Abhängigkeit von eines Orts;
- Verwenden des berechneten zweiten Geschwindigkeitsprofils zum Aktualisieren des physiologischen Parameters des Auges.

17. Verfahren nach einem der Ansprüche 13 bis 16, wobei das erste Geschwindigkeitsprofil mindestens eines umfasst von: einer Geschwindigkeit in dem räumlichen Bereich, einer Geschwindigkeit in dem Zeitbereich.

18. Verfahren nach einem der Ansprüche 16 bis 17, wobei das zweite Geschwindigkeitsprofil mindestens eines umfasst von: der Geschwindigkeit in dem räumlichen Bereich, der Geschwindigkeit in dem Zeitbereich.

**Revendications**

1. Appareil (100) de détermination de paramètre physiologique d'un œil, l'appareil comprenant ;

- une sonde magnétique allongée (102, 610, 702), ayant une première extrémité (102A), une seconde extrémité (102B) opposée à la première extrémité et une section médiane (102C) entre la première extrémité et la seconde extrémité ;
- une bobine d'entraînement (104) agencée partiellement pour entourer la sonde magnétique allongée
- une bobine de mesure (106, 602, 708, 1004) comprenant au moins une première section (106A) et une deuxième section (106B), et la bobine de mesure étant agencée partiellement pour entourer la sonde magnétique allongée et
- un dispositif de commande (108) configuré pour
- alimenter sélectivement la bobine d'entraînement afin de créer une force magnétique pour initier le mouvement de la sonde magnétique allongée en direction de la première extrémité, dans lequel la sonde magnétique allongée est dirigée pour frapper une surface de l'œil et rebondir sur celle-ci ;
- mesurer des valeurs de première tension induite sur la première section et des valeurs de tension induite commune sur au moins l'une parmi : la première section et la deuxième section, ou sur la deuxième section, en fonction du temps pendant une durée du mouvement de la sonde magnétique allongée ;
- déterminer des valeurs de localisateur en fonction du temps en divisant les valeurs de première tension induite par les valeurs de tension induite commune ;
- mettre en correspondance les valeurs de localisateur en fonction du temps du domaine temporel avec un domaine spatial et
- calculer, à partir des valeurs de localisateur de domaine spatial, un premier profil de vitesse de la sonde magnétique allongée, et utiliser le premier profil de vitesse calculé de la sonde magnétique allongée afin de déterminer le paramètre physiologique de l'œil.

2. Appareil (100) selon la revendication 1, dans lequel la bobine de mesure (106, 602, 708, 1004) est utilisée pendant une première période de temps en tant que bobine d'entraînement (104) et pendant une seconde période de temps, qui est la seconde période de temps après la première période de temps, en tant que deuxième section de la bobine de mesure.

3. Appareil (100) selon l'une quelconque des revendications précédentes, dans lequel, lors de l'utilisation, la première section de la bobine de mesure (106, 602, 708, 1004) entoure une première section de la sonde magnétique allongée (102, 610, 702) et la deuxième section de la bobine de mesure entoure une deuxième section de la sonde magnétique allongée, dans lequel la première section de la sonde magnétique allongée est différente de la deuxième section de la sonde magnétique allongée, lorsque la sonde magnétique allongée est dans sa première position spatiale.

4. Appareil (100) selon la revendication 3, dans lequel, lors de l'utilisation, la première section de la bobine de mesure (106, 602, 708, 1004) n'entoure pas la première section de la sonde magnétique allongée (102, 610, 702) et la deuxième section de la bobine de mesure entoure la deuxième section de la sonde magnétique allongée, lorsque la sonde magnétique allongée se trouve dans sa seconde position spatiale, laquelle deuxième position spatiale est différente de la première position spatiale.

5. Appareil (100) selon l'une quelconque des revendications précédentes, dans lequel le domaine temporel au domaine spatial est mis en correspondance par au moins l'une parmi : une fonction de transfert prédéterminée ou une table de consultation.

6. Appareil (100) selon l'une quelconque des revendications précédentes, dans lequel la première section et la deuxième section de la bobine de mesure (106, 602, 708, 1004) sont connectées en série.

7. Appareil (100) selon l'une quelconque des revendications précédentes, dans lequel la bobine de mesure (106, 602, 708, 1004) comprend une troisième section, la troisième section étant connectée en série avec la première et la deuxième section de la bobine de mesure et la troisième section est agencée pour entourer une troisième section de la sonde magnétique allongée (102, 610, 702).

8. Appareil (100) selon l'une quelconque des revendications précédentes, dans lequel la tension induite commune est mesurée sur toutes les sections de la bobine de mesure (106, 602, 708, 1004).

9. Appareil (100) selon l'une quelconque des revendications précédentes, dans lequel le dispositif de commande (108) est en outre configuré pour

- mesurer une valeur de seconde tension induite à partir d'une section autre que la première section, en fonction du temps pendant une durée du mouvement de la sonde magnétique allongée (102, 610, 702) ;
- déterminer une valeur de second localisateur en fonction du temps en divisant les valeurs de seconde tension induite par les valeurs de tension induite commune ;
- mettre en correspondance la fonction de second localisateur d'un domaine temporel avec un domaine spatial ;
- calculer, à partir des valeurs de second localisateur de domaine spatial, un second profil de vitesse de la sonde magnétique allongée, et
- utiliser le second profil de vitesse calculé de la sonde magnétique allongée afin de mettre à jour le paramètre physiologique de l'œil.

10. Appareil (100) selon l'une quelconque des revendications précédentes, dans lequel le premier profil de vitesse comprend au moins l'une parmi : une vitesse dans le domaine spatial, une vitesse dans le domaine temporel.

11. Appareil (100) selon l'une quelconque des revendications 9 à 10 précédentes, dans lequel le second profil de vitesse comprend au moins l'une parmi : une vitesse dans le domaine spatial, une vitesse dans le domaine temporel.

12. Appareil (100) selon l'une quelconque des revendications précédentes, dans lequel l'appareil est un tonomètre.

13. Procédé de détermination d'un paramètre physiologique d'un œil, le procédé utilisant un dispositif de commande afin d'effectuer les étapes de procédé comprenant :

- l'alimentation d'une bobine d'entraînement (104) pour déplacer une sonde magnétique allongée (102, 610, 702) dans une direction d'une première extrémité (102A) de la sonde magnétique allongée, dans lequel la sonde magnétique allongée est dirigée pour frapper une surface de l'œil et rebondir sur celle-ci ;
- la mesure en fonction du temps, sur une première section d'une bobine de mesure (106, 602, 708, 1004), d'une première tension induite ;
- la mesure en fonction du temps, sur au moins l'une parmi : une première section et une deuxième section ou sur la deuxième section de la bobine de mesure, d'une tension commune induite ;
- la détermination d'une valeur de premier localisateur en fonction du temps en divisant les valeurs de première tension induite mesurées par les valeurs de tension commune induite mesurées respectives ;
- la mise en correspondance des valeurs de premier localisateur du domaine temporel avec un domaine spatial ; - le calcul, à partir des valeurs de premier localisateur de domaine spatial, d'un premier profil de vitesse de la sonde magnétique allongée, et
- l'utilisation du premier profil de vitesse afin de déterminer le paramètre physiologique de l'œil.

14. Procédé selon la revendication 13, dans lequel les mesures sont effectuées pendant le mouvement de la sonde magnétique allongée (102, 610, 702) afin d'obtenir des valeurs de tension induite en fonction du temps.

15. Procédé selon l'une quelconque des revendications 13 à 14, dans lequel la détermination du paramètre physiologique de l'œil est effectuée au moins par l'une parmi : l'analyse de l'accélération de la sonde magnétique allongée (102, 610, 702) pendant son impact sur la surface du corps du patient, de la variation de vitesse avant l'impact et après l'impact, de la quantité de pénétration de la sonde magnétique allongée sur la surface du corps.

16. Procédé selon l'une quelconque des revendications 13 à 15, dans lequel la valeur de paramètre physiologique de l'œil est mise à jour par

- la mesure en fonction du temps, avec une section d'une bobine de mesure (106, 602, 708, 1004) différente de la première section, d'une seconde
première tension induite ;
- la détermination d'une valeur de second localisateur en fonction du temps en divisant les valeurs de seconde tension induite par les valeurs de tension induite commune ;
- la mise en correspondance de la fonction de second localisateur d'un domaine temporel avec un domaine spatial ;
- le calcul, à partir des valeurs de second localisateur de domaine spatial, d'un second profil de vitesse de la sonde magnétique allongée (102, 610, 702) en fonction de l'emplacement ;
- l'utilisation du second profil de vitesse calculé afin de mettre à jour le paramètre physiologique de l'œil.

17. Procédé selon l'une quelconque des revendications 13 à 16, dans lequel le premier profil de vitesse comprend au moins l'une parmi : une vitesse dans le domaine spatial, une vitesse dans le domaine temporel.

18. Procédé selon l'une quelconque des revendications 16 à 17, dans lequel le second profil de vitesse comprend au moins l'une parmi : la vitesse dans le domaine spatial, la vitesse dans le domaine temporel.

FIG. 1

EP 4 380 426 B1

FIG. 2

FIG. 3

**—●— U23-U12    —◆— U34-U23    —■— U45-U34**

## FIG. 4

Z(mm)

502

## FIG. 5

FIG. 6

FIG. 7

FIG. 8

**FIG. 9**

FIG. 10

EP 4 380 426 B1

FIG. 11A

FIG. 11B

Signal voltages of coil halves
vs.probe position in mm.

FIG. 12A

U+=U35+U13, U-=U35-U13.

FIG. 12B

Driver coil 0.2V

FIG. 13A

| U+ | U- | 1000*(U+/U-) |
|---|---|---|
| 559.2 | 387.09 | 692.2 |
| 574.7 | 311.52 | 542.1 |
| 596.3 | 223.52 | 374.8 |
| 599.4 | 128.15 | 213.8 |
| 599.8 | -13.431 | -22.4 |
| 573.1 | -124.19 | -216.7 |
| 539.5 | -266.42 | -493.8 |
| 487.6 | -371.36 | -761.6 |

FIG. 13B

Driver coil 0.3 volts

$y = -255,97x + 548,63$

--●-- U+  —●— U-  —●— U-/U+  ·········· Linear (U-/U+)

## FIG. 13C

| U+ | U- | 1000*(U-/U+) |
|---|---|---|
| 622.3 | 163.3 | 242.41 |
| 616.6 | 39.2 | 63.57 |
| 606.8 | -116.8 | -192.49 |
| 587.4 | -287 | -488.59 |
| 588.5 | -436.3 | -741.38 |
| 590.6 | -514.85 | |
| 570.3 | -529.11 | |
| 532.1 | -507.34 | |

## FIG. 13D

ENERGIZE DRIVER COIL TO MOVE ELONGATED MAGNETIC PROBE
TO DIRECTION OF FIRST END OF ELONGATED MAGNETIC PROBE
1402

MEASURE AS FUNCTION OF TIME, WITH FIRST SECTION OF
MEASUREMENT COIL, INDUCED FIRST VOLTAGE
1404

MEASURE AS FUNCTION OF TIME, OVER MEASUREMENT COIL,
INDUCED COMMON VOLTAGE
1406

DETERMINE FIRST LOCATOR VALUES AS FUNCTION OF TIME BY
DIVIDING MEASURED INDUCED FIRST VOLTAGE VALUES WITH
RESPECTIVE MEASURED INDUCED COMMON VOLTAGE VALUES
1408

A

FIG. 14A

(A)

MAP FIRST LOCATOR VALUES FROM TIME DOMAIN TO SPATIAL DOMAIN
1410

CALCULATE FROM SPATIAL DOMAIN FIRST LOCATOR VALUES FIRST VELOCITY PROFILE OF ELONGATED MAGNETIC PROBE
1412

USE FIRST VELOCITY PROFILE TO DETERMINE PHYSIOLOGICAL PARAMETER
1414

# FIG. 14B

Locator, Derivative and Speed function

FIG. 15

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2008103381 A1
- US 2019380577 A1